(19) Europäisches Patentamt
European Patent Office
Office européen des brevets

(11) Numéro de publication : **0 288 352 B1**

(12) # FASCICULE DE BREVET EUROPEEN

(45) Date de publication du fascicule du brevet : **18.09.91 Bulletin 91/38**

(21) Numéro de dépôt : **88400849.1**

(22) Date de dépôt : **08.04.88**

(51) Int. Cl.$^5$ : **C07C 219/22**, C07D 295/08, C07D 217/04, C07D 211/14, C07D 233/60, A61K 31/22, A61K 31/415, A61K 31/44, A61K 31/47, A61K 7/00, A01N 37/12

(54) **Dérivés aromatiques, leur préparation et leur utilisation comme antimicrobiens.**

(30) Priorité : **10.04.87 FR 8705165**

(43) Date de publication de la demande : **26.10.88 Bulletin 88/43**

(45) Mention de la délivrance du brevet : **18.09.91 Bulletin 91/38**

(84) Etats contractants désignés : **AT BE CH DE ES FR GB GR IT LI LU NL SE**

(56) Documents cités :
**DE-A- 1 445 968**
**FR-A- 2 137 722**
**FR-M- 3 525**
**FR-M- 3 660**
**GB-A- 863 223**
**GB-A- 1 434 826**
**US-A- 3 767 810**

(73) Titulaire : **SANOFI**
**40, Avenue George V**
**F-75008 Paris (FR)**

(72) Inventeur : **Mosse, Madeleine La Chanterelle**
**Chemin du Réservoir de Montmaur**
**F.34100 Montpellier (FR)**
Inventeur : **Proietto, Vincenzo**
**1 Cours du Merle**
**F-34680 Saint Georges D'Orques (FR)**

(74) Mandataire : **Gillard, Marie-Louise et al**
**Cabinet Beau de Loménie 55, Rue d'Amsterdam**
**F-75008 Paris (FR)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet de nouveaux dérivés aromatiques substitués par un groupement (oméga amino) alcanol estérifié.

La présente invention concerne également l'utilisation des composés selon l'invention dans des compositions à usage antiseptique, antimicrobien, comme désinfectants ou conservateurs notamment dans les domaines de la pharmacie, la cosmétologie ou l'agroalimentaire.

Par un autre de ses aspects, la présente invention se réfère au procédé de préparation des composés selon l'invention.

Plus précisemment, la présente invention a pour objet de nouveaux dérivés aromatiques de formule :

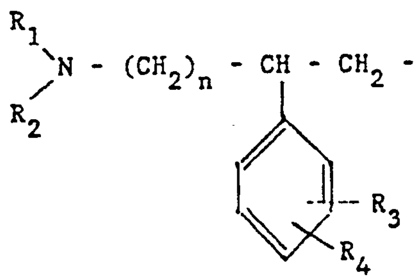

(I)

dans laquelle :

— n représente un nombre entier compris entre 2 et 10 ;

— $R_1$ et $R_2$ sont identiques ou différents et représentent un groupe cycloalkyle de 3 à 6 atomes de carbone, un alkyle comportant de 1 à 6 atomes de carbone,non substitué ou substitué par un groupement phényle ou benzyle ;
ou $R_1$ et $R_2$ forment ensemble avec l'atome d'azote auquel ils sont liés un hétérocycle choisi parmi les groupes pyrrolidinyl-1, pipéridino, azépinyl-1, hexaméthylèneimino, méthyl-4 pipéridino, benzyl-4-pipéridino, phényl-4 pipéridino, tétrahydro-1,2,3,4 isoquinolyl-2, morpholino, imidazolyl-1 ;

— $R_3$ représente un hydrogène, un halogène, un méthyle ou un phényle ;

— $R_4$ représente un hydrogène, un halogène ou un méthyle ;
ou $R_3$ et $R_4$, pris ensemble avec le noyau benzénique auquel ils sont liés, constituent un groupement naphtyl-1 ou naphtyl-2 ;

— Y représente une liaison directe, un groupement méthylèneoxy, un groupement méthylènethio ou un groupement vinylène ;

— $R_5$ représente un alkyle comportant de 5 à 18 atomes de carbone, un cycloalkyle comportant de 3 à 8 atomes de carbone, un groupe adamantyle, naphtyl-1, naphtyl-2 ou phényle non substitué ou substitué par un ou 2 substituants choisis parmi les atomes d'halogènes, le groupe trifluorométhyle, le groupe nitro ou le groupe phényle ;
ainsi que leurs sels avec les acides minéraux ou organiques.

Plus précisemment, la présente invention se réfère à des composés de formule (I) dans laquelle $R_3$ représente un hydrogène, un halogène, un méthyle ou un phényle et $R_4$ représente un hydrogène, un halogène ou un méthyle.

La présente invention se réfère également à des composés de formule (I) dans laquelle $R_3$ et $R_4$, pris ensemble avec le noyau benzénique auquel ils sont liés, constituent un groupement naphthyl-1 ou naphtyl-2.

Le dichloro-2,4 benzoate de diméthylamino-5 (naphtyl-1-)-2 pentyle et ses sels sont des composés préférés selon l'invention.

Par halogène, on désigne dans la présente description le chlore, le brome, l'iode ou le fluor.

Le procédé selon l'invention est caractérisé en ce que l'on estérifie l'aminoalcool de formule :

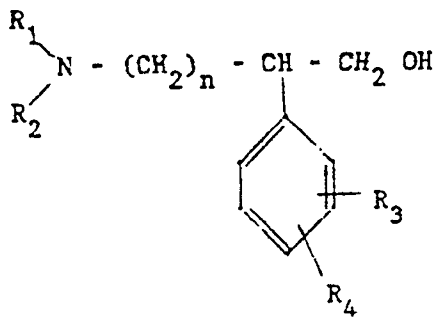

(II)

dans laquelle $R_1$, $R_2$, $R_3$, $R_4$, et n sont tels que définis ci-dessus, ou un de ses sels avec un acide minéral ou organique, par un acide ou un halogénure d'acide de formule :

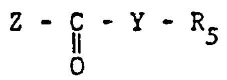

(III)

dans laquelle Y et $R_5$ sont tels que définis ci-dessus et Z représente un atome d'halogène ou le groupement OH.

La réaction est effectuée à une température pouvant varier entre 50°C et 120°C soit en utilisant l'acide ou l'halogénure d'acide comme solvant, soit dans un solvant non réactif tel que le dichloroéthane ou la pyridine.

Lorque la réaction est effectuée sur un sel de l'aminoalcool (II), on prépare le composé (I) selon l'invention également sous forme de sel.

L'aminoalcool (II) est préparé par réduction de l'ester ou de l'acide correspondant de formule :

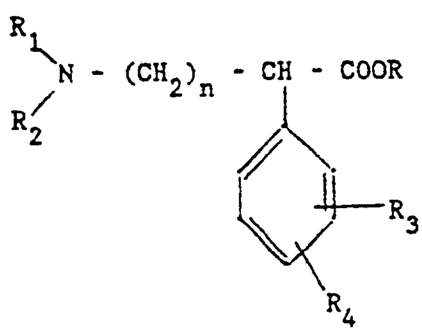

(IV)

dans laquelle $R_1$, $R_2$, $R_3$ et $R_4$ sont tels que définis ci-dessus et R représente l'hydrogène ou un groupe alkyle.

Cette réduction est réalisée par des moyens connus tels que l'action d'un agent réducteur ou une réaction d'électrolyse en milieu acide. Comme agent réducteur, on utilise un hydrure métallique, éventuellement en présence d'un catalyseur. Plus particulièrement, la réduction de l'acide ou de l'ester (IV) pour obtenir l'aminoalcool (II), peut être réalisée par l'hydrure de bore, par l'hydrure d'aluminium, par le borohydrure de lithium, par le borohydrure d'aluminium, par le borohydrure de sodium, par l'hydrure de sodium et d'aluminium, par l'hydrure de lithium et d'aluminium ou par un autre hydrure du bore tel que le diméthylsulfure de borane, ou le benzodioxaborole-1,2,3. De façon préférentielle, la réduction est réalisée sur l'acide (R = H) ou sur l'ester éthylique ($R = C_2H_5$) par action du Vitride$^R$ (hydrure de sodium bis (méthoxy-2 éthoxy) aluminium) dans un solvant inerte tel que le benzène ou le toluène à une température comprise entre la température ambiante et 80°C. Le composé obtenu est isolé selon les méthodes habituelles, par exemple par simple précipitation.

Les acides et leurs esters (IV) sont préparés par des méthodes connues. On utilise comme produit de départ du phénylacétonitrile substitué sur le noyau benzénique par $R_3$ et $R_4$ ou le naphtyl-1 acétonitrile ou le naphtyl-2 acétonitrile, selon le composé attendu, sur lequel on fait agir de l'amidure de sodium dans un solvant inerte porté à reflux et on ajoute une chloroalkylamine de formule :

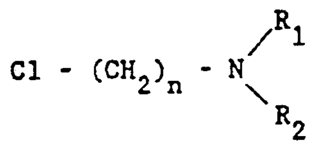 (V) ;

on chauffe au reflux du solvant pour former le composé :

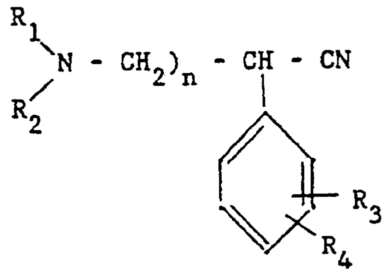 (VI) .

On hydrolyse le nitrile (VI) en milieu acide fort (pH inférieur à 2) pour obtenir l'acide correspondant qui est éventuellement estérifié par un groupe alkyle R selon des méthodes connues.

Les halogénures d'acide ou les acides correspondants (III) sont connus.

Les exemples suivants illustrent l'invention sans toutefois la limiter.

EXEMPLE 1 :

Chlorhydrate de dichloro-2,4 benzoate de [diméthylamino-4 (naphtyl-1)-2 butyle] : SR 44083 A.

A) Chlorhydrate de diméthylamino-4 (naphtyl-1)-2 butanol.

a) Diméthylamino-4 (naphtyl-1)-2 butyronitrile.

On ajoute par petites fractions 6,9 g d'amidure de sodium à 29,4 g de naphtyl-1 acétonitrile dans 250 ml de benzène anhydre et on chauffe à 80°C pendant 2 heures. On refroidit à + 40°C et on ajoute goutte à goutte 19 g de chloro-2 diméthylaminoéthane puis on chauffe à nouveau à 80°C pendant 4 heures. On refroidit et ajoute 200 ml d'eau. Après décantation, la phase organique est extraite par une solution d'acide chlorhydrique à 10%. La phase aqueuse est lavée à l'éther, neutralisée par une solution de soude à 10%, extraite à l'éther, puis lavée à l'eau et séchée sur sulfate de sodium. Après évaporation du solvant, on obtient 24 g du produit attendu.

b) Acide diméthylamino-4 (naphtyl-1)-2 butyrique.

On chauffe au reflux pendant 2 heures 19 g du produit obtenu à l'étape précédente dans 150 ml d'une solution acide acétique/acide sulfurique/eau (1/1/1 en volume). On refroidit, dilue à l'eau et lave 2 fois à l'éther. La phase aqueuse est neutralisée par une solution de soude à 30%, lavée à l'éther, puis la phase aqueuse est à nouveau acidifiée par de l'acide chlorhydrique concentré et évaporée à sec. On extrait le résidu à l'éthanol. Après évaporation de l'éthanol, on recristallise dans un mélange éthanol/éther éthylique. On obtient 9 g de produit attendu.

Point de fusion : 130-135°C avec décomposition.

c) Chlorydrate de diméthylamino-4 (naphtyl-1)-2 butanol.

5,1 g du produit obtenu précédemment sont ajoutés par petites fractions à 11,8 g de Vitride[R]/ hydrure de sodium bis (méthoxy-2 éthoxy) aluminium / à 70% dans du toluène. On chauffe à 80-90°C pendant 3 heures puis on refroidit à +10°C et on verse sur l'eau.

On extrait à l'éther, lave à l'eau et sèche sur sulfate de sodium. On reprend le résidu à l'éther éthylique sec, ajoute de l'éther chlorhydrique et évapore à sec sous vide. On reprend ensuite par de l'alcool isopropylique sec et chauffe à reflux pour solubiliser. On refroidit puis ajoute goutte à goutte de l'éther éthylique anhydre. Le

précipité formé est filtré puis lavé plusieurs fois à l'éther anhydre et séché puis recristallisé dans un mélange éthanol/éther isopropylique. On obtient 3,3 g du produit attendu.

Rendement : 60% ;

Point de fusion : 163°C.

1.1.SR 44083 A.

2,9 g de l'alcool préparé à l'étape précédente et 10 ml de chlorure de dichloro-2,4 benzoyle sont chauffés à 100°C pendant une demi-heure. On refroidit, précipite sur éther, filtre, lave à l'éther, sèche sous vide et recristallise dans un mélange alcool isopropylique/éther éthylique. On obtient 4 g du produit attendu.

Rendement : 87% ;

Point de fusion : 147-150°C.

1.2 EXEMPLE 2 :

Chlorhydrate de dichloro-2,4 benzoate de [(dichloro-3,4 phényl)-2 hexaméthylèneimino butyle] : SR 44102 A.

1.2. Chlorhydrate de (dichloro-3,4 phényl)-2 hexaméthylèneimino-4 butanol.

1.2.a)1(Dichloro-3,4 phényl)-2 hexaméthylèneimino-4 butyronitrile.

On ajoute par petites portions 4 g d'amidure de sodium à 16,5 g de dichloro-3,4 phénylacétonitrile dans 200 ml d'éther anhydre et on chauffe 2 heures au reflux : On ajoute ensuite, goutte à goutte, 16,10 g de N-héxaméthylèneimino chloro-2 éthane et chauffe 5 heures au reflux, on refroidit et ajoute 200 ml d'eau, décante la phase organique, extrait avec 300 ml d'acide chlorhydrique à 10%. On lave la phase aqueuse à l'éther, neutralise avec de la soude à 30% et extrait à l'éther. On lave la phase organique à l'eau saturée en chlorure de sodium, sèche sur sulfate de magnésium puis on évapore le solvant pour obtenir 26 g du produit attendu.

Rendement : 84%.

1.2.b)2(Dichloro-3,4 phényl)-2 hexaméthylèneimino-4 butanoate de méthyle.

On chauffe à reflux pendant 2 heures 20 g du produit obtenu à l'étape précédente dans 90 ml d'une solution eau/acide sulfurique/acide acétique (1/1/1 en volume). On refroidit, verse sur 200 ml d'eau et lave à l'éther. On ajoute de la soude à 30% à la phase aqueuse pour atteindre pH = 10, on lave à l'éther et acidifie à pH = 1 avec de l'acide chlorhydrique. On évapore l'eau et reprend les résidus avec du méthanol à chaud. On évapore la moitié du méthanol, ajoute quelques gouttes d'acide sulfurique puis on chauffe 5 heures au reflux. On élimine le solvant, reprend l'huile restante à l'eau et neutralise avec de la soude à 30%. On extrait à l'éther, lave à l'eau saturée en chlorure de sodium, sèche sur sulfate de magnésium puis évapore à sec. On obtient 15,1 g de produit attendu.

Rendement : 76,5%.

1.2.c)3(Dichloro-3,4 phényl)-2 hexaméthylèneimino-4 butanol.

On ajoute goutte à goutte à 14,5 g du produit obtenu à l'étape précédente 12,6 g de Vitride® (hydrure de sodium bis (méthoxy-2 éthoxy) aluminium) à 70% dans du toluène à 0°C. On laisse réagir 30 minutes à température ambiante. On verse sur l'eau, extrait à l'éther, lave à l'eau saturée en chlorure de sodium, sèche sur sulfate de magnésium puis évapore le solvant. Le résidu est repris dans l'éther chlorhydrique. Après évaporation du solvant, le produit est repris dans 20 ml d'alcool isopropylique puis on verse goutte à goutte de l'éther anhydre jusqu'à ce que le produit cristallise. Le précipité est lavé à l'éther, séché sous vide. On obtient 12 g du produit.

Rendement 81% ;

Point de fusion 155°C.

1.2.SR 44102 A.

On chauffe à 100°C 1,5 g de l'alcool obtenu à l'étape précédente dans 10 ml de chlorure de dichloro-2,4 benzoyle pendant 30 minutes. On précipite ensuite sur éther, filtre, lave à l'éther, sèche sous vide et recristallise

dans un mélange éther/alcool isopropylique. On obtient 1,9 g du produit attendu.
Rendement : 90% ;
Point de fusion : 179-181°C.

EXEMPLE 3 :

Chlorhydrate de p-chloro cinnamate de [(dichloro-3,4 phényl)-2 diméthylamino-5 pentyle] : SR 44429 A.

A) Chlorhydrate de (dichloro-3,4 phényl)-2 diméthylamino-5 pentanol.

a) (Dichloro-3,4 phényl)-2 diméthylamino-5 valéronitrile.

3,9 g d'amidure de sodium sont ajoutés par petites portions à 18,6 g de dichloro-3,4 phénylacétonitrile en solution dans 200 ml d'éther anhydre. On chauffe 2 heures à reflux, on ajoute 12,1 g de chloro-3 diméthylamino propane et on continue le chauffage à reflux pendant 2 heures et demie. On refroidit, ajoute de l'eau et décante la phase organique. On lave à l'eau et extrait avec de l'acide chlorhydrique à 10%. On neutralise ensuite la phase aqueuse avec de la soude à 30%, extrait à l'éther et évapore le solvant. On obtient 17 g de produit attendu.
Rendement : 62,5%.

b) (Dichloro-3,4 phényl)-2 diméthylamino-5 pentanoate de méthyle.

17 g du produit obtenu précédemment sont chauffés à reflux pendant 5 heures avec 75 ml d'une solution acide sulfurique/acide acétique/eau (1/1/1 en volume). On refroidit, verse sur de l'eau et lave à l'éther. On neutralise la phase aqueuse avec de la soude à 30% et sépare l'huile qui décante. Celle-ci est lavée à l'éther puis acidifiée par de l'acide chlorhydrique à 35%. On reprend alors par du méthanol et quelques gouttes d'acide sulfurique puis on chauffe à reflux pendant une nuit. Après refroidissement, on évapore le méthanol, neutralise par du bicarbonate de sodium et extrait à l'éther. On obtient 8 g de produit.

c) Chlorhydrate de (dichloro-3,4 phényl)-2 diméthylamino-5 pentanol.

8 g de l'ester obtenu à l'étape précédente sont réduits par 6,8 g de Vitride® à 70% dans le toluène à température ambiante. On verse sur l'eau glacée, extrait à l'éther, lave à l'eau, sèche sur sulfate de magnésium et évapore à sec. Le résidu est repris à l'éther puis on ajoute de l'éther chlorhydrique. On filtre, sèche et recristallise dans l'éther. On obtient 8 g du produit attendu.
Rendement 98% ;
Point de fusion 115-117°C.

B) SR 44429 A.

On porte à reflux pendant 3 heures 3,1 g du produit obtenu à l'étape précédente et 4,02 g de chorure de p-chloro cinnamoyle dans 50 ml de dichloroéthane. On refroidit, évapore à sec, reprend par un minimum de dichloroéthane et précipite à l'éther. On obtient 3 g du produit attendu que l'on recristallise dans un mélange éthanol/éther.
Rendement : 64% ;
Point de fusion : 136-137°C.

EXEMPLE 4 :

Chlorhydrate de dichloro-2,4 phénoxyacétate de [benzyl-4 pipéridino)-4 (naphtyl-1)-2 butyle] : SR 44541 A.

A) Chlorhydrate de (benzyl-4 pipéridino)-4 (naphtyl-1)-2 butanol.

a) (Benzyl-4 pipéridino)-4 (naphtyl-1)-2 butyronitrile.

On ajoute par petites portions 2 g d'amidure de sodium à une solution de naphtyl-1 acétonitrile dans 150 ml d'éther anhydre et on chauffe à reflux pendant 5 heures. On ajoute alors 11,8 g de (benzyl-4 pipéridino)-2 chloroéthane et on chauffe à nouveau à reflux pendant 3,5 heures. On ajoute 200 ml d'eau, décante la phase

organique, extrait par 300 ml d'acide chlorhydrique à 10%. La phase aqueuse est lavée à l'éther, neutralisée par de la soude à 30% et extraite à l'éther. On lave la phase organique à l'eau saturée en chlorure de sodium et sèche sur sulfate de magnésium puis on évapore le solvant. On obtient 16,4 g de produit attendu.

b) (Benzyl-4 pipéridino)-4 (naphtyl-1)-2 butanoate de méthyle.

On chauffe à reflux pendant 2 heures 14 g du produit obtenu à l'étape précédente dans 75 ml d'une solution eau/acide sulfurique/acide acétique (1/1/1 en volume).

On refroidit, verse sur 200 ml d'eau et lave à l'éther. On ajoute de la soude à 30% à la phase aqueuse jusqu'à environ pH = 10, on lave à l'éther, acidifie à pH = 1 par de l'acide chlorhydrique puis on évapore l'eau et reprend les résidus avec du méthanol à chaud. Après évaporation de la moitié du méthanol on ajoute quelques gouttes d'acide sulfurique puis on chauffe 5 heures à reflux. On élimine le solvant, reprend l'huile restante à l'eau et neutralise par de la soude à 30%. On extrait à l'éther, lave à l'eau saturée en chlorure de sodium, sèche sur sulfate de magnésium et évapore à sec. On obtient 13,7 g de produit attendu.

Rendement 87% ;

c) Chlorhydrate de (benzyl-4 pipéridino)-4 (naphtyl-1)-2 butanol.

On ajoute goutte à goutte 13 g du produit précédemment obtenu à 9,7 g de Vitride® dans du toluène à 0°C.

On laisse réagir 30 minutes à température ambiante puis on verse sur l'eau, extrait à l'éther, lave à l'eau saturée en chlorure de sodium, et sèche sur sulfate de magnésium. On évapore à sec puis le produit est repris dans l'éther chlorhydrique. Après évaporation, le produit est repris dans 20 ml d'éthanol puis précipité dans l'éther. Le précipité est lavé à l'éther puis séché sous vide. On obtient 8 g de produit attendu.

Rendement : 61% ;

Point de fusion : 185-186°C.

B) SR 44541 A

On porte à reflux pendant 2 heures dans 50 ml de dichloroéthane 2,1 g du produit préparé à l'étape précédente et 5 g de chlorure de dichloro-2,4 phényloxyacétyle. On réduit le volume de moitié par évaporation sous vide puis on précipite à l'éther, filtre, lave à l'éther et sèche sous vide. On obtient 2,7 g de produit qui sont recristallisés dans l'éther.

Rendement : 90% ;

Point de fusion : 92°C.

EXEMPLES 5 à 40

En utilisant les mêmes méthodes que précédemment, on a préparé les produits selon l'invention qui sont décrits dans le tableau III ci-dessous. Ils sont caractérisés par leur point de fusion (Fc) après recristallisation dans un solvant. Les solvants de recristallisation (solvant) sont utilisés purs ou en mélange équivolumique. La signification des abréviations est la suivante :

- Alcool méthylique : MeOH ;
- Alcool éthylique : EtOH ;
- Alcool isopropylique : iPrOH ;
- Ether éthylique : $Et_2O$ ;
- Ether isopropylique : $(iPr)_2O$ ;
- Dichlorométhane : DCM ;
- Dichloroéthane : DCE.

Les produits obtenus sous forme huileuse sont caractérisés par leur spectres de RMN enregistrés dans le DMSO à 250 MHz

Les déplacements chimiques (delta) sont mesurés en p.p.m. ; les abréviations suivantes sont utilisées

- s pour singulet
- d pour doublet
- t pour triplet
- m pour multiplet .

TABLEAU III

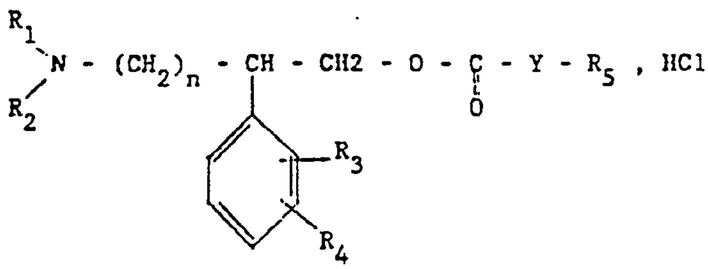

| Ex. n° | Produit SR | $NR_1R_2$ | n | 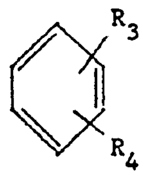 | -Y- | $R_5$ | $F_c$ °C solvant |
|---|---|---|---|---|---|---|---|
| 5 | 44026A | héxaméthylèneimino | 2 | naphtyl-2 | - | 2,4 - Cl phényl | 179-182 $EtOH/Et_2O$ |
| 6 | 44085A | diméthylamino | 2 | naphtyl-1 | - | phényl | 133-136 $iPrOH/Et_2O$ |
| 7 | 44086A | 1,2,3,4-tétrahydro isoquinolyl | 2 | naphtyl-1 | - | 2,4 - Cl phényl | 198-200 $iPrOH/Et_2O$ |
| 8 | 44099A | héxaméthylèneimino | 2 | 2,4 - Cl phényl | - | " | 144-146 $MeOH/Et_2O$ |
| 9 | 44100A | " | 2 | " | - | 4 - Cl phényl | 181-184 $MeOH/Et_2O$ |

| | | | | | | | |
|---|---|---|---|---|---|---|---|
| 10 | 44101A | héxaméthylèneimino | 2 | " | - | 4 - F phényl | 182-183 iPrOH/$Et_2O$ |
| 11 | 44149A | pipéridino | 2 | naphtyl-1 | - | 4 - Cl phényl | 194-196 iPrOH/$Et_2O$ |
| 12 | 44150A | " | 2 | " | - | 3 - Cl phényl | 88-91 iPrOH/$Et_2O$ |
| 13 | 44151A | " | 2 | " | - | 2 - Cl phényl | 132-134 $(iPr)_2O$ |
| 14 | 44152A | " | 2 | " | - | 2,4 - Cl phényl | 187-190 EtOH/$Et_2O$ |
| 15 | 44194A | imidazolyl | 2 | " | - | " | 130-134 iPrOH/$Et_2O$ |
| 16 | 44225A | diisopropylamino | 2 | " | - | " | 169-172 iPrOH/$Et_2O$ |
| 17 | 44384A | diméthylamino | 2 | " | CH=CH | " | 133-134 DCE/$Et_2O$ |
| 18 | 44385A | " | 2 | " | - | 3 - $CF_3$ phényl | 152-154 $Et_2O$ |
| 19 | 44417A | pipéridino | 3 | biphénylyl | - | 2,4 - Cl phényl | 128-130 $Et_2O$ |

| | | | | | | | |
|---|---|---|---|---|---|---|---|
| 20 | 44418A | pipéridino | 3 | " | - | " | 141-143 $Et_2O$ |
| 21 | 44427A | diméthyl- amino | 2 | naphtyl-1 | $CH_2S$ | phényl | 113 $Et_2O$ |
| 22 | 44428A | " | 3 | 3,4 - Cl phényl | - | 2,4 - Cl phényl | 117 EtOH/$Et_2O$ |
| 23 | 44430A | " | 2 | naphtyl-1 | CH=CH | 4 - Cl phényl | 182 EtOH/$Et_2O$ |
| 24 | 44431A | héxaméthy- lèneimino | 2 | 3,4 - Cl phényl | CH=CH | 2,4 - Cl phényl | 200-202 EtOH/$Et_2O$ |
| 25 | 44433A | " | 2 | " | - | cyclo- héxyl | 210-212 EtOH/$Et_2O$ |
| 26 | 44537A | morpholino | 2 | 2,4 - Cl phényl | - | biphé- nylyl | 180 iPrOH |
| 27 | 44538A | benzyl-4 pipéridino | 2 | naphtyl-1 | - | phényl | 134 $Et_2O$ |
| 28 | 44539A | morpholino | 2 | 2,4 - Cl phényl | - | 2,4 - Cl phényl | 138 iPrOH/$Et_2O$ |
| 29 | 44540A | " | 2 | " | - | naphtyl-1 | 176 iPrOH |

| | | | | | | | |
|---|---|---|---|---|---|---|---|
| 30 | 44603A | diméthyl-amino | 3 | naphtyl-1 | - | 2,4 - Cl phényl | 128-130 DCM/$Et_2O$ |
| 31 | 44661A | diméthyl-amino | 3 | naphtyl-1 | - | 4 - F phényl | 103-105 iPrOH/$Et_2O$ |
| 32 | 44699A | " | 3 | naphtyl-1 | - | 4 - I phényl | 184-185 iPrOH/$Et_2O$ |
| 33 | 44700A | " | 3 | naphtyl-1 | - | $C_{10}H_{23}$ | 71-73 EtO2 |
| 34 | 44888A | diéthyl-amino | 3 | 4-Br phényl | CH=CH | 4 - Cl phényl | 129-130 iPrOH/$Et_2O$ |
| 35 | 44945A | diméthyl-amino | 6 | naphtyl-1 | - | 2,4 - Cl phényl | huileux (RMN) |
| 36 | 45026A | diéthyl-amino | 6 | naphtyl-1 | - | 2,4 - Cl phényl | huileux (RMN) |
| 37 | 45029A | diméthyl-amino | 6 | naphtyl-1 | - | $C_{10}H_{23}$ | huileux (RMN) |
| 38 | 45031A | " | 6 | 4 - Br phényl | - | 2,4 - Cl phényl | 55-58 iPrOH/$Et_2O$ |
| 39 | 45179A | " | 3 | 4 - Br phényl | - | 2,4 - Cl phényl | 116-118 iPrOH/$Et_2O$ |

| | | | | | | | |
|---|---|---|---|---|---|---|---|
| 40 | 45262A | diméthyl-amino | 3 | naphtyl-1 | - | 3,5-$NO_2$ phényl | 218-220 $Et_2O/EtOH$ |
| 41 | 45349A | " | 3 | naphtyl-1 | - | $(CH_2)_{16}$-$CH_3$ | 66-68 $Et_2O$ |
| 42 | 45350A | " | 3 | " | - | $(CH_2)_4$ - $CH_3$ | 87-89 $Et_2O$ |

SPECTRES DE RMN

SR 44945 A :
(Exemple 35)

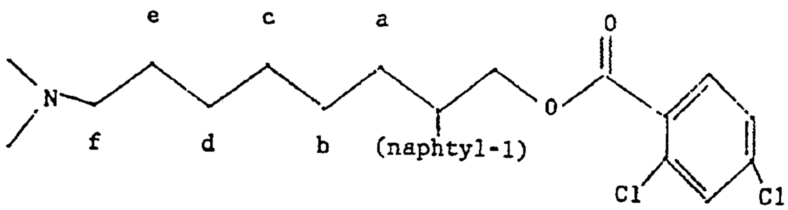

, HCl

| Delta p.p.m. | Aspect | Protons | Attribution |
|---|---|---|---|
| 1,18 | m | 6H | $CH_2$ a,b,c |
| 1,5 | m | 2H | $CH_2$ d |
| 1,85 | m | 2H | $CH_2$ e |
| 2,59 | s | 6H | $(CH_3)_2$ N |
| 2,61 | s | | |

| | | | | |
|---|---|---|---|---|
| 2,85 | m | 2H | $CH_2$ f | |
| 4 | m | 1H | C<u>H</u> $CH_2$ OCO | |
| 4,5 | d J=7Hz | 2H | CH $CH_2$ OCO | |
| 7,3-8,3 | massif | 10H | H aromatiques | |

SR 45026 A :

(Exemple 36)

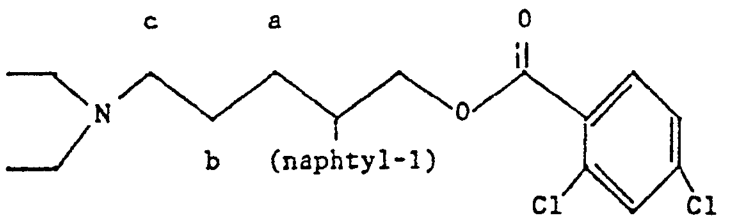

, HCl

| Delta p.p.m. | Aspect | Protons | Attribution |
|---|---|---|---|
| 1,05 | m | 6H | $2CH_3$ (Ethyl) |
| 1,58 | m | 2H | $CH_2$ a |
| 1,92 | m | 2H | $CH_2$ b |
| 2,92 | m | 6H | $2CH_2$ (Ethyl) + $CH_2$ c |

| | | | |
|---|---|---|---|
| 4,05 | m | 1H | $C\underline{H}\ CH_2\ OCO$ |
| 4,5 | d J=7Hz | 2H | $CH\ C\underline{H_2}\ OCO$ |
| 7,3-8,3 | massif | 10H | H aromatiques |

SR 45029 A
(Exemple 37)

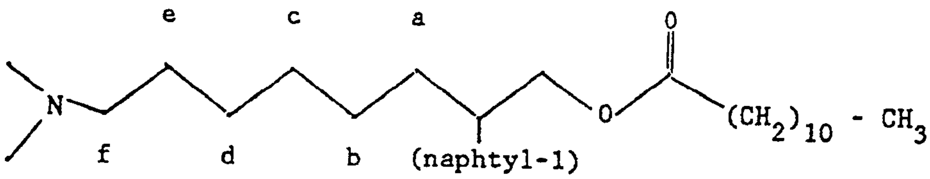

| Delta p.p.m. | Aspect | Protons | Attribution |
|---|---|---|---|
| 0,8 | t j=6Hz | 3H | $C\underline{H_3}\ (CH_2)_{10}$ |
| 0,9-1,5 | massif | 26H | $CH_2$ a,b,c,d, $CH_2-(\underline{CH_2})_9-CH3$ |
| 1,75 | m | 2H | $CH_2$ c |
| 2-2,3 | massif | 10H | $(CH_3)_2\ N$ + $CH_2\ CO$ + $CH_2$ f |
| 3,8 | m | 1H | $C\underline{H}\ CH_2\ OCO$ |

| | | | |
|---|---|---|---|
| 4,22 | m | 2H | CH $CH_2$ OCO |
| 7,3-8,2 | massif | 7H | H aromatiques |

L'activité bactéricide des produits selon l'invention a été étudiée sur différentes souches par la méthode décrite ci-après :

Un inoculum bactérien est mis en contact avec différentes dilutions du produit à tester, et ce pendant un temps limité : 30 minutes. A la fin du contact, une partie aliquote du mélange suspension bactérienne/produit est déposée à la surface d'un milieu de culture gélosé contenant un neutralisant de l'activité antibactérienne du produit. La concentration bactéricide retenue est la concentration minimale de produit à partir de laquelle les bactéries ne poussent plus. Cette concentration est exprimée en µg/ml.

Les souches bactériennes choisies pour l'étude sont :

1 — Escherichia Coli CNCM 54125 ;
2 — Klebsiella pneumoniae capsulée RO30 ;
3 — Pseudomonas aeruginosa CNCM A22 ;
4 — Streptococcus faecalis CNCM 5855 ;
5 — Staphylococcus aureus CNCM 53154.

La deuxième souche est entretenue sur milieu de Worgel Fergusson, les autres sur Tryptic Soy Agar-Difco (TSA), commercialisé par Difco.

Après 24 heures de culture à 37°C, on récolte la pousse microbienne à l'aide de billes de verre et de 10 ml de diluant contenant 1 g de tryptone et 8,5 g de chlorure de sodium dans 1000 ml d'eau distillée. On agite la suspension formée et on mesure au spectrophotomètre le pourcentage de transmission de la lumière à 620 nm :

— Souche 1 : 70% ;
— Souche 2 : 80% ;
— Souche 3 : 70% ;
— Souche 4 : 60% ;
— Souche 5 : 60%.

L'inoculum bactérien correspond à une dilution au 1/20 ème de cette suspension bactérienne.

Des plaques comportant des cupules reçoivent différentes dilutions du produit à étudier. Ces dilutions du produit à étudier sont mises en contact avec les différentes suspensions bactériennes à l'aide d'un inoculateur à site multiple. Après 20 minutes de contact, des parties aliquotes sont transférées à l'aide de cet inoculateur à la surface d'un milieu gélosé (TSA) placé dans des boîtes de Petri, contenant un neutralisant de l'activité, à savoir 20 g de Lubrol W, 25 g de Tween 80 et 2,5 g de thiosulfate de sodium dans 1000 ml de TSA (Difco). Un témoin de l'efficacité du neutralisant est réalisé pour chaque produit étudié en déposant à la surface du milieu de culture une partie aliquote de la dilution du produit à étudier. Après séchage, l'inoculum correspondant est déposé au même endroit. Un témoin inoculum correspondant est déposé au même endroit. Un témoin inoculum est réalisé sur milieu gélosé avec et sans neutralisant. La lecture se fait après 48 heures d'incubation à 37°C.

Les résultats obtenus avec les composés des exemples identifiés à la fois par leur numéro d'exemple et la référence interne du déposant (SR) sont rassemblés dans le tableau I ci-dessous.

TABLEAU I

Concentration minimale bactéricide (CMB) en µg/ml

| Ex. n° | Numéro du Produit | Souches bactériennes 1 | 2 | 3 | 4 | 5 |
|---|---|---|---|---|---|---|
| 5 | SR 44026 A | 10 | 50 | 50 | 10 | 10 |
| 1 | SR 44083 A | 5 | 10 | 50 | 10 | 50 |
| 6 | SR 44085 A | 50 | 50 | 50 | 50 | 100 |
| 7 | SR 44086 A | 50 | 50 | 100 | 50 | 500 |
| 8 | SR 44099 A | 5 | 50 | 50 | 5 | 50 |
| 9 | SR 44100 A | 5 | 50 | 50 | 10 | 100 |
| 10 | SR 44101 A | 5 | 10 | 50 | 10 | 200 |
| 2 | SR 44102 A | 5 | 50 | 50 | 5 | 10 |
| 11 | SR 44149 A | 10 | 10 | 50 | 10 | 50 |
| 12 | SR 44150 A | 10 | 50 | 50 | 50 | 50 |
| 13 | SR 44151 A | 50 | 50 | 100 | 50 | 500 |
| 14 | SR 44152 A | 10 | 10 | 100 | 10 | 50 |
| 15 | SR 44194 A | 50 | 50 | 50 | 50 | 500 |
| 16 | SR 44225 A | < 10 | 50 | 100 | < 10 | 500 |
| 17 | SR 44384 A | 10 | 10 | 10 | 10 | 10 |
| 18 | SR 44385 A | 10 | 50 | 50 | 10 | 50 |
| 19 | SR 44417 A | 5 | 5 | 50 | 5 | 5 |
| 20 | SR 44418 A | 10 | 10 | 50 | 50 | 50 |
| 21 | SR 44427 A | 50 | 50 | 50 | 50 | 500 |
| 22 | SR 44428 A | 5 | 5 | 10 | 10 | 10 |
| 3 | SR 44429 A | 5 | 5 | 10 | 5 | 5 |
| 23 | SR 44430 A | 5 | 5 | 10 | 5 | 10 |
| 24 | SR 44431 A | 1 | 5 | 5 | 5 | 5 |
| 25 | SR 44433 A | 50 | 50 | 50 | 50 | 500 |
| 26 | SR 44537 A | 50 | - | 100 | 50 | 500 |
| 27 | SR 44538 A | 10 | - | 100 | 50 | 100 |
| 28 | SR 44539 A | 50 | - | 100 | 50 | 500 |
| 29 | SR 44540 A | 50 | - | 100 | 50 | 500 |
| 4 | SR 44541 A | 10 | - | 50 | 50 | 50 |
| 30 | SR 44603 A | 5 | - | 5 | 10 | 10 |
| 31 | SR 44661 A | 50 | - | 50 | 50 | 50 |
| 32 | SR 44699 A | 5 | - | 50 | 5 | 200 |
| 33 | SR 44700 A | 2 | - | 5 | 2 | 2 |
| 34 | SR 44888 A | 10 | - | 10 | 10 | 10 |
| 35 | SR 44945 A | 5 | - | 5 | 5 | 5 |
| 36 | SR 45026 A | 10 | - | 50 | 10 | 200 |
| 37 | SR 45029 A | 10 | - | 10 | 5 | 10 |
| 38 | SR 45031 A | 5 | - | 10 | 5 | 10 |
| 39 | SR 45179 A | 10 | - | 50 | 50 | 50 |
| 40 | SR 45262 A | 50 | - | 200 | 50 | 50 |

Les résultats montrent que les produits selon l'invention présentent un large spectre d'activité sur les souches bactériennes testées. Cette activité bactéricide s'exprime dans un temps court (inférieur ou égal à 30 minutes).

L'activité antifongique des produits selon l'invention a également été déterminée en utilisant la méthode décrite précédemment.

Une souche représentative des levures a été choisie pour l'étude : Candica albicans CNCM 1180.

Elle est entretenue sur milieu gélosé de Sabouraud Dextrose Agar, commercialisé par Difco, la technique est identique à celle décrite pour l'étude de l'activité antibactérienne. Après 48 heures de culture à 37°C, on récolte la pousse microbienne à l'aide de billes de verre et de 5 ml de diluant contenant 1 g de tryptone et 8,5 g de chlorure de sodium dans 1000 ml d'eau distillée : 5 ml du diluant sont ensuite rajoutés. Cette suspension donne au spectromètre un pourcentage de transmission de la lumière à 620 nm de 2 à 3%. Une dilution au 1/100 de cette suspension, observée entre lame et lamelle à l'objectif 40 d'un microscope, doit montrer 10 cellules par champ, ce qui correspond à 1000000 levures par ml.

Les résultats, obtenus avec des composés selon les exemples sont rassemblés dans le tableau II ci-dessous.

TABLEAU II

Concentration minimale fongicide (CMF) en µg/ml

| Ex. n° | Produit | CMF |
|---|---|---|
| 5 | SR 44026 A | 50 |
| 1 | SR 44083 A | 10 |
| 6 | SR 44085 A | 100 |
| 7 | SR 44099 A | 50 |
| 2 | SR 44102 A | 50 |
| 11 | SR 44149 A | 100 |
| 12 | SR 44150 A | 100 |
| 17 | SR 44384 A | 50 |
| 24 | SR 44431 A | 10 |
| 26 | SR 44537 A | 100 |
| 27 | SR 44538 A | 100 |
| 28 | SR 44539 A | 100 |
| 29 | SR 44540 A | 100 |
| 4 | SR 44541 A | 50 |
| 30 | SR 44603 A | 10 |
| 31 | SR 44661 A | 200 |
| 32 | SR 44699 A | 50 |
| 33 | SR 44700 A | 2 |
| 34 | SR 44888 A | 50 |
| 35 | SR 44945 A | 5 |
| 37 | SR 45029 A | 10 |
| 38 | SR 45031 A | 10 |
| 39 | SR 45179 A | 50 |
| 40 | SR 45262 A | 200 |

Ces résultats montrent que les produits selon l'invention possèdent une activité antifongique intéressante et rapide.

La tolérance des produits selon l'invention a été étudiée chez le cobaye. Les animaux sont tondus de part et d'autre de la ligne médiane du dos, la tonte est entretenue tous les 2 jours. Des lots de 6 animaux reçoivent sur la zone tondue 0,2 ml d'une solution aqueuse ou alcoolique du produit selon l'invention. Lorsque les produits sont en solution alcoolique, un lot témoin d'animaux reçoit l'alcool sur un côté.

Pour l'étude de la tolérance cutanée, le traitement est appliqué 1 fois par jour, 6 jours sur 7, durant 3 semaines. Les observations sur le plan cutané portent sur la présence d'érythème, d'éruption cutanée ou d'hyperkératose dont l'intensité est graduée selon un barème déterminé.

L'essai de sensibilisation cutanée est réalisé sur les mêmes animaux après deux semaines de repos. Le

traitement dure une semaine, il est identique au précédent. L'évaluation se fait selon les mêmes critères et d'après le même barème que celui utilisé pour la tolérance locale.

On a constaté que les produits selon l'invention sont bien tolérés lorsqu'ils sont appliqués à des concentrations allant jusqu'à 2%. De plus ils ne présentent aucun effet sensibilisant.

Une évaluation de la toxicité aiguë par voie orale a été réalisée chez la souris. Cette étude a été effectuée sur des souris mâles de souche CD1 provenant de l'élevage Charles River. Chaque lot était composé de 5 animaux d'un poids corporel variant de 24 à 30 g entretenus dans une même cage. Les animaux étaient conservés à jeun pendant 6 heures avant le traitement. Pour chaque étude, le produit, mis en suspension dans une solution de gomme arabique à 10%, a été administré par gavage à l'aide d'une sonde oesophagienne. La nourriture était de nouveau distribuée aux animaux 4 heures après le gavage et les animaux étaient gardés en observation pendant une période de 14 jours après l'administration. Pendant cette période, on note la mortalité dans chacun des lots mis en expérience et, lorsque cela est possible, on détermine la dose létale 50 ($DL_{50}$) en utilisant la méthode de J.T. LITCHFIELD et R. WILCOXON, J. Pharmacol. 1949, 95, 99-113. On a constaté que la $DL_{50}$ per os des produits selon l'invention est supérieure à 1000 mg/kg.

Les produits selon l'invention, qui présentent une bonne activité antimicrobienne, peuvent être utilisés dans des préparations pharmaceutiques, désinfectantes, cosmétiques ou alimentaires, notamment comme antiseptiques par voie locale et générale, comme désinfectants et comme conservateurs.

En tant qu'antiseptiques à usage humain ou vétérinaire, la concentration en produit actif peut varier de 0,01% à 5% selon l'usage et la formulation choisie. Ainsi on peut préparer des solutions moussantes détergentes destinées au lavage des mains du chirurgien et du personnel soignant ou à la détersion de lésions dermatologiques telles qu'impétigo, pityriasis, ulcères des jambes. Des solutions moussantes détergentes servent également comme shampoings (antipelliculaires par exemple) ou pour la préparation de gels pour douches, de crèmes à raser, de lotions moussantes. On obtient des solutions moussantes contenant des produits selon l'invention en utilisant des tensioactifs amphotères, anioniques, cationiques ou non ioniques à une concentration de 0,3 à 30%, des agents humectants tels que les glycols ou des polyéthylèneglycols de 0 à 20%, des copolymères d'oxyde d'éthylène et de polypropylène de 0 à 20%, un alcool (éthanol, isopropanol, alcool benzylique) ou un polyol tel que le glycérol de 0 à 15%, ainsi que des complexants des ions Ca ++, Mg ++, des métaux lourds, des sels apportant un pouvoir tampon approprié, des agents viscosants tels que NaCl ou KCl, des polymères naturels, cellulosiques ou synthétiques tels que le polyvinylpyrrolidone, des surgraissants épaississants tels que le distéarate de polyéthylèneglycol, le mono- ou di-éthanolamide de coprah, des parfums, conservateurs, colorants.

Lorsque le produit selon l'invention est difficilement soluble dans l'eau, on pourra utiliser des microémulsions, des solutions micellaires ou toute autre phase du diagramme ternaire ou quaternaire eau/principe actif/tensioactif/cotensioactif, permettant la solubilisation dans l'eau. Ces solutions peuvent être diluées ou non, elles peuvent par exemple être distribuées à l'aide d'une vasopompe ou de gaz pulseurs liquéfiés ou non.

En utilisant les mêmes constituants à des concentrations appropriées, on peut également préparer, avec les produits selon l'invention, des solutions aqueuses simples ou sous forme de sprays destinés à l'antisepsie des champs opératoires, aux soins post-opératoires, soins des brûlés, eczémas surinfectés, érythèmes fessiers, plaies, acné, ou destinés à des déodorants.

Des solutions alcooliques simples ou sous forme de sprays contenant 20 à 80% d'alcool peuvent comporter, outre les excipients utilisés dans les solutions aqueuses, des excipients permettant de franchir les couches kératinisées de la peau et des phanères tels que Azone (commercialisé par Nelson Research) et Transcutol (commercialisé par Gattefossé). Ces solutions sont destinées à l'antisepsie de la peau avant ponctions, la préparation du champ opératoire, l'antisepsie des mains du personnel soignant, aux soins des dermatoses infectées fermées, folliculites, perionyxis ou acné.

Les produits selon l'invention peuvent être appliqués sous forme de crèmes qui contiennent certains des composés cités pour la préparation des solutions ainsi que les corps gras habituellement rencontrés dans la préparation des crèmes ou émulsions. Ces crèmes sont utilisables notamment pour la prévention de surinfections de l'érythème fessier, de l'eczéma, des mycoses ou de l'acné.

Les produits selon l'invention peuvent également être utilisés pour le traitement ou la prévention des maladies sexuellement transmissibles sous forme d'ovules, comprimés gynécologiques ou éponges gynécologiques ou en complément de préservatifs. Les ovules peuvent contenir de 0 à 99% de triglycérides, polyéthylèneglycols de différents poids moléculaires, tweens, polymères naturels ou synthétiques, polyols, savons. Les comprimés gynécologiques peuvent contenir des diluants tels que lactose ou cellulose, des lubrifiants tels que le stéarate de magnésium, des agents d'écoulement tels que la silice, des agents de désagrégation tels que le carboxyméthylamidon ou la cellulose.

Les produits selon l'invention peuvent être administrés sous forme de sprays avec embouts nasal et buccal dans les syndromes infectieux des voies respiratoires (rhinites, sinusites, angines, amygdalites, pharingites)

ou sous forme de gels ou de bains de bouche pour le traitement des gingivites, pyorrhées ou la prévention de la plaque dentaire, dans ce cas on pourra également utiliser des dentifrices contenant les produits selon l'invention. Les formes destinées à l'administration buccale ou nasale peuvent contenir les mêmes excipients que les solutions auxquelles on ajoute éventuellement des aromatisants pour la sphère buccale ou les constituants nécessaires à l'isotonicité pour les sprays nasaux ; les dentifrices contiennent en outre des silices colloïdales pyrogénées ou non, du carbonate de calcium, des édulcorants et des sels de fluor.

Les produits selon l'invention peuvent être utilisés dans des collyres, solutions oculaires ou pommades ophtalmiques pour le traitement des infections de l'oeil (blépharites ou conjonctivites par exemple) ou en liquide de rinçage des lentilles cornéennes. Pour préparer ces formes oculaires, on peut utiliser les mêmes constituants que ceux utilisés pour les solutions en veillant à assurer l'isotonicité du mélange.

De plus, les produits selon l'invention peuvent être administrés par voie générale chez l'homme par exemple par voie orale, sous forme de gélules, comprimés ou comprimés entériques comme antiseptiques intestinaux.

Les produits selon l'invention peuvent également être utilisés chez l'animal dans des indications telles que la prévention ou le traitement des lésions infectées ou susceptibles de se surinfecter. Les compositions pharmaceutiques sont alors similaires à celles utilisées chez l'homme, en particulier des crèmes, sprays ou solutions.

Par ailleurs, l'action létale rapide sur les germes des produits selon l'invention permet de les utiliser comme désinfectants de surface à des concentrations pouvant varier de 0,1 à 4%. Les produits sont alors mis en oeuvre dans des préparations telles que des solutions moussantes détergentes, aqueuses ou non aqueuses, des sprays ou nébulisations. De telles préparations sont particulièrement utiles dans les domaines hospitaliers ou vétérinaires, pour les collectivités locales ou les industries agroalimentaires. Ces préparations peuvent contenir les mêmes constituants que ceux utilisés dans les formulations à usage antiseptique, on peut toutefois y adjoindre des solvants organiques divers.

Enfin, l'activité antimicrobienne de ces produits permet de les utiliser comme conservateurs dans les industries parmaceutique, cosmétique et alimentaire. Les produits selon l'invention sont alors utilisés comme additifs aux formulations pharmaceutiques, cosmétiques ou alimentaires à des concentrations pouvant varier de 0,005 à 0,5%. On peut également utiliser ces composés comme additifs désinfectants dans les peintures.

Différentes formulations des produits selon l'invention peuvent être préparées selon l'application choisie.

EXEMPLE 43 :

Préparation antiseptique liquide détergente moussante.

| | | |
|---|---|---|
| SR 44 102 A | | 0,5 g |
| Paraffine sulfonate de sodium | | 15 g |
| Hydroxyde de sodium ou | | |
| acide lactique | qsp pH 5,2 | |
| Eau purifiée | qsp | 100 g |

EXEMPLE 44 :

Soluté antiseptique alcoolique.

| | | | |
|---|---|---|---|
| SR 44 384 A | | 0,2 | g |
| Alkyldiméthylcarboxyméthylamine (solution à 30 %) | | 0,5 | g |
| Condensat d'oxyde d'éthylène et de propylène glycol L 62 | | 1 | g |
| Acide lactique ou hydroxyde de sodium | qsp pH 6,5 | | |
| Alcool éthylique à 70° | qsp | 100 | g. |

EXEMPLE 45 :

Préparation antiseptique liquide détergente moussante.

| | | | |
|---|---|---|---|
| SR 44 083 A | | 0,1 | g |
| Alkyldiméthylcarboxyméthylamine (solution à 30 %) | | 15 | g |
| Tétracémate disodique | | 0,1 | g |
| Propylène glycol | | 20 | g |
| Hydroxyde de sodium | qsp pH 5,8 | | |
| Eau purifiée | qsp | 100 | g |

EXEMPLE 46 :

Collutoire.

| | | | |
|---|---|---|---|
| SR 44 083 A | | 0,3 | g |
| Alcool éthylique à 95° | | 14 | g |
| Essence d'anis | | 0,00225 | ml |
| Eugénol | | 0,00075 | ml |
| Glycérine | | 20 | ml |
| Saccharine | | 0,03 | g |
| Hydroxyde de sodium soluté | qsp pH 5,5 | | |
| Eau purifiée | qsp | 100 | ml. |

EXEMPLE 47 :

Ovules antiseptiques destinés au traitement des maladies sexuellement transmissibles.

| | |
|---|---|
| SR 44 431 A | 500 mg |
| Mélange eutectique d'esters d'acides gras | 2,568 g. |

Comme mélange eutectique d'esters d'acides gras, on peut utiliser Suppocire A R commercialisé par Gattefossé.

EXEMPLE 48 :

Collyre.

| | |
|---|---|
| SR 44 603 A | 0,2 g |
| Chlorure de sodium | 1,4 g |
| Eau pour préparations injectables | qsp 100 ml. |

EXEMPLE 49 :

Comprimés entériques.

| | |
|---|---|
| SR 44 384 A | 200 mg |
| Hydroxypropylméthyl cellulose 6 cP | 6 mg |
| Lactose | 114 mg |
| Cellulose microcristalline | 60 mg |
| Carboxyméthylamidon sodique | 12 mg |
| Stéarate de magnésium | 8 mg |
| Pour un comprimé nu terminé à | 400 mg |

Enrobage

| | |
|---|---|
| Eudragit L 100 | 0,9 mg |
| Phtalate dibutyle | 0,9 mg |
| Acétone | 14,1 mg |
| Alcool isopropylique | 14,1 mg |
| Comprimé enrobé terminé à | 430 mg. |

EXEMPLE 50 :

Spray.

| | | |
|---|---|---|
| SR 44 026 A | | 2 g |
| Ethanol à 95° | | 20 g |
| Propylène glycol | | 5 g |
| Hydroxyde de Na | qsp pH 5,5 | |
| Eau | qsp | 100 g |
| Agent pulseur | qs | |

EXEMPLE 51 :

Spray filmogène.

| | | |
|---|---|---|
| SR 44 083 A | | 0,5 g |
| Polyvinylpyrrolidone | | 2 g |
| Résine acrylique | | 2 g |
| Ethanol à 95° | qsp | 100 g |
| Agent pulseur | qs | |

EXEMPLE 52 :

Un produit selon l'invention peut être utilisé comme conservateur dans une crème émulsion.

| | | |
|---|---|---|
| Huile de vaseline | | 6 g |
| Mélange d'alcool cétostéarylique et d'alcool cétostéarylique oxyéthyléné | | 9 g |
| Phosphate monosodique anhydre | | 0,300 g |
| Tétracémate disodique | | 0,010 g |
| Vaseline | | 15 g |
| SR 44 429 A | | 0,100 g |
| Acide phosphorique | qsp pH 4,5 | |
| Eau purifiée | qsp | 100 g. |

EXEMPLE 53 :

Le produit selon l'invention peut être utilisé comme conservateur dans une crème pour utilisation cosmétologique.

| | | |
|---|---|---|
| Collagène | 0,500 | g |
| Carboxypolyméthylène 934 | 0,400 | g |
| Lanoline hydrogénée | 4 | g |
| Perhydrosqualène | 20 | g |
| Monopalmitate de sorbitol polyoxyéthyléné | 2 | g |
| SR 44 428 A | 0,150 | g |
| Acide lactique ou hydroxyde de sodium qsp pH 6,5 | | |
| Eau purifiée qsp | 100 | g. |

EXEMPLE 54 :

Conservateur dans une huile antisolaire.

| | | |
|---|---|---|
| Huile minérale 65/75 | 68 | g |
| Huile de ricin | 8 | g |
| Huile de sésame | 20 | g |
| Alcool isopropylique | 2 | g |
| Eusolex® | 1,5 | g |
| Parfum | 0,4 | g |
| SR 44 430 A | 0,100 | g. |

(Eusolex® est commercialisé par Merck)

EXEMPLE 55 :

Un produit selon l'invention peut être utilisé comme conservateur dans un shampoing.

| | | |
|---|---|---|
| Palmitate de potassium et d'acides aminés | 20 | g |
| Alkylsulfates de sodium | 2 | g |
| Diéthanolamide de coprah | 5 | g |
| Acétate de linalyle | 0,200 | g |
| SR 44 431 A | 0,05 | g |
| Hydroxyde de sodium qsp pH 7 | | |
| Eau purifiée qsp | 100 | g. |

EXEMPLE 56 :

Conservateur pour jus de fruits ou confiture.

SR 44417 A micronisé 0,02%.

EXEMPLE 57 :

Désinfectant pour surfaces inertes.

| | | |
|---|---|---|
| SR 44 026 A | | 2 g |
| Dodécyldiméthylcarboxydiméthylamine | | 20 g |
| Tétracémate disodique | | 2 g |
| Acide lactique | qsp pH 3,5 | |
| Eau purifiée | qsp | 100 g |

**Revendications**

**Revendications pour les Etats Contractants suivants : AT, BE, CH, DE, FR, GB, IT, LI, LU, NL, SE**

1. Composé de formule :

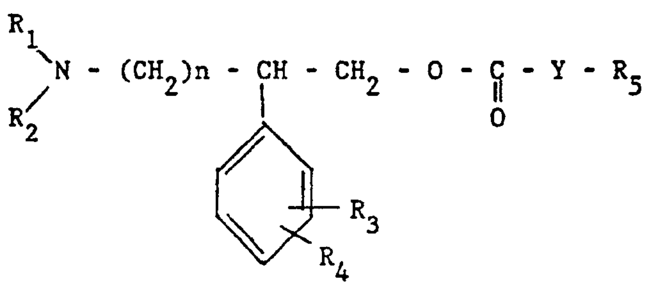

(I)

dans laquelle :

— n est un nombre entier compris entre 2 et 10 ;

— $R_1$ et $R_2$ sont identiques ou différents et représentent un cycloalkyle de 3 à 6 atomes de carbone, un alkyle comportant de 1 à 6 atomes de carbone non substitué ou substitué par un groupement phényle ou benzyle ;

ou $R_1$ et $R_2$ forment ensemble avec l'atome d'azote auquel ils sont liés un hétérocycle choisi parmi les groupes pyrrolidinyl-1, pipéridino, azépinyl-1, hexaméthylèneimino, méthyl-4 pipéridino, benzyl-4 pipéridino, phényl-4 pipéridino, tétrahydro-1,2,3,4 isoquinolyl-2, morpholino, imidazolyl-1 ;

— $R_3$ représente un hydrogène, un halogène, un méthyle ou un phényle ;

— $R_4$ représente un hydrogène, un halogène ou un méthyle ;

ou $R_3$ et $R_4$ pris ensemble avec le noyau benzénique auquel ils sont liés constituent un groupement naphtyl-1 ou naphtyl-2 ;

— Y représente une liaison directe, un groupement méthylèneoxy, un groupement méthylènethio ou un groupement vinylène ;

— $R_5$ représente un alkyle comportant de 5 à 18 atomes de carbone, un cycloalkyle comportant de 3 à 8 atomes de carbone, un groupe adamantyle, naphtyl-1, naphtyl-2 ou phényle non substitué ou substitué par un ou 2 substituants choisis parmi les atomes d'halogènes, le groupe trifluorométhyle, le groupe nitro ou le groupe phényle ;

ainsi que leurs sels avec les acides minéraux ou organiques.

2. Composé selon la revendication 1, caractérisé en ce que $R_3$ représente un hydrogène, un halogène, un méthyle ou un phényle et $R_4$ représente un hydrogène, un halogène ou un méthyle.

3. Composé selon la revendication 1, caractérisé en ce que $R_3$ et $R_4$ pris ensemble avec le noyau benzénique auquel ils sont liés constituent un groupement naphtyl-1 ou naphtyl-2.

4. Le dichloro-2,4 benzoate de diméthylamino-5 (naphtyl-1)-2 pentyle et ses sels.

5. Procédé pour la préparation d'un composé selon l'une quelconque des revendications 1 à 4, caractérisé en ce que l'on estérifie l'aminoalcool de formule :

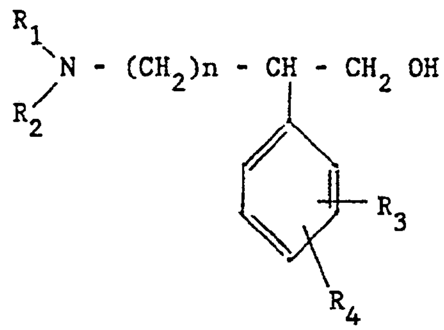

(II)

dans laquelle $R_1$, $R_2$, $R_3$, $R_4$ et n sont tels que définis dans la revendication 1, ou un de ses sels avec un acide minéral ou organique, par un acide ou un halogénure d'acide de formule :

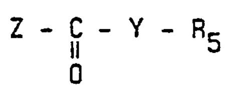

(III)

dans laquelle Y et $R_5$ sont tels que définis dans la revendication 1 et Z représente le groupement OH ou un halogénure.

6. Utilisation d'un composé selon l'une quelconque des revendications 1 à 4, dans des préparations pharmaceutique, désinfectante, cosmétique ou alimentaire.

7. Composition pharmaceutique présentant une activité antimicrobienne et désinfectante, caractérisée en ce qu'elle contient de 0,01 à 5% du composé selon l'une quelconque des revendications 1 à 4.

8. Composition désinfectante pour surfaces inertes, caractérisée en ce qu'elle contient de 0,1 à 4% du composé selon l'une quelconque des revendications 1 à 4.

9. Composition pharmaceutique caractérisé en ce qu'elle contient à titre de conservateur de 0,005 à 0,5% du composé selon l'une quelconque des revendications 1 à 4

10. Produit cosmétique, caractérisé en ce qu'il contient à titre de conservateur de 0,005 à 0,5% du composé selon l'une quelconque des revendications 1 à 4.

11. Produit alimentaire, caractérisé en ce qu'il contient à titre de conservateur de 0,005 à 0,5% du composé selon l'une quelconque des revendications 1 à 4.

**Revendications pour l'Etat Contractant suivant : ES**

1. Procédé pour l'obtention de dérivés aromatiques répondant à la formule :

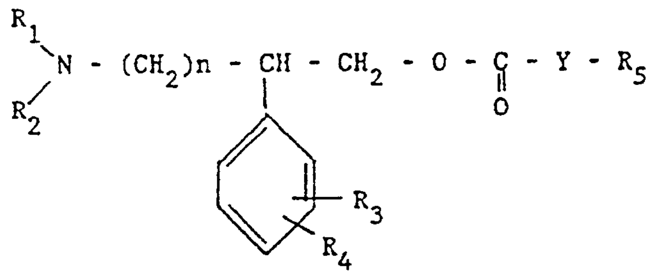

(I)

dans laquelle :

— n est un nombre entier compris entre 2 et 10 ;

— $R_1$ et $R_2$ sont identiques ou différents et représentent un cycloalkyle de 3 à 6 atomes de carbone, un un alkyle comportant de 1 à 6 atomes de carbone non substitué ou substitué par un groupement phényle ou benzyle ;

ou $R_1$ et $R_2$ forment ensemble avec l'atome d'azote auquel ils sont liés un hétérocycle choisi parmi les groupes pyrrolidinyl-1, pipéridino, azépinyl-1, hexaméthylèneimino, méthyl-4 pipéridino, benzyl-4 pipéridino, phényl-4 pipéridino, tétrahydro-1,2,3,4 isoquinolyl-2, morpholino, imidazolyl-1 ;

— $R_3$ représente un hydrogène, un halogène, un méthyle ou un phényle ;

— $R_4$ représente un hydrogène, un halogène ou un méthyle ;

ou $R_3$ et $R_4$ pris ensemble avec le noyau benzénique auquel ils sont liés constituent un groupement naphtyl-1 ou naphtyl-2 ;

— Y représente une liaison directe, un groupement méthylèneoxy, un groupement méthylènethio ou un groupement vinylène ;

— $R_5$ représente un alkyle comportant de 5 à 18 atomes de carbone, un cycloalkyle comportant de 3 à 8 atomes de carbone, un groupe adamantyle, naphtyl-1, naphtyl-2 ou phényle non substitué ou substitué par un ou 2 substituants choisis parmi les atomes d'halogènes, le groupe trifluorométhyle, le groupe nitro ou le groupe phényle,

caractérisé en ce que l'on estérifie l'aminoalcool de formule :

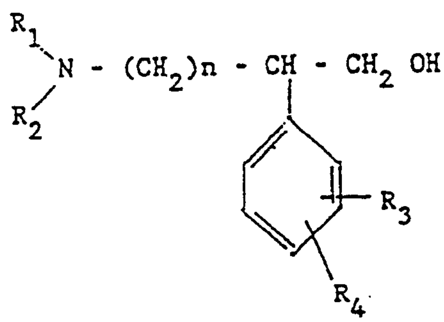

(II)

dans laquelle $R_1$, $R_2$, $R_3$, $R_4$ et n sont tels que définis ci-dessus, ou un des ses sels avec un acide minéral ou organique, par un acide ou un halogénure d'acide de formule :

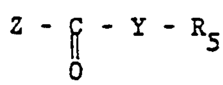

(III)

dans laquelle Y et $R_5$ sont tels que définis ci-dessus et Z représente le groupement OH ou un halogénure.

2. Procédé selon la revendication 1, caractérisé en ce que l'on utilise un aminoalcool de formule (II), dans laquelle $R_3$ représente un hydrogène, un halogène, un méthyle ou un phényle et $R_4$ représente un hydrogène, un halogène ou un méthyle.

3. Procédé selon la revendication 1, caractérisé en ce que l'on utilise un aminoalcool de formule (II) dans laquelle $R_3$ et $R_4$ pris ensemble avec le noyau benzénique auquel ils sont liés constituent un groupement naphtyl-1 ou naphtyl-2.

4. Procédé selon la revendication 1, caractérisée en ce que l'on utilise comme aminoalcool le composé

de formule II, dans lequel $R_1$ et $R_2$ sont le groupe méthyle, n est 3 et $R_3$ et $R_4$ forment, avec le groupe benzénique auxquels ils sont liés le groupe naphtyl-1 et en ce qu'on fait réagir ledit aminoalcool avec un composé de formule III dans laquelle Z est tel que défini dans la revendication 1, Y est une liaison directe et $R_5$ est le groupe 2,4-dichlorophényle.

5. Utilisation d'un composé de formule (I) obtenu par le procédé selon l'une quelconque des revendications 1 à 4, comme antiseptique, désinfectant, conservateur, dans des préparations désinfectante, cosmétique ou alimentaire.

6. Utilisation d'un composé de formule (I) obtenu par le procédé selon l'une quelconque des revendications 1 à 4, en association avec un véhicule pharmaceutiquement acceptable pour la préparation de compositions pharmaceutiques.

7. Utilisation selon la revendication 6, pour la préparation de compositions pharmaceutiques présentant une activité antimicrobienne et désinfectante contenant de 0,01 à 5% du composé de formule (I).

8. Composition désinfectante pour surfaces inertes, caractérisée en ce qu'elle contient de 0,1 à 4% du composé selon l'une quelconque des revendications 1 à 4.

9. Composition caractérisée en ce qu'elle contient à titre de conservateur de 0,005 à 0,5% du composé selon l'une quelconque des revendications 1 à 4.

10. Produit cosmétique, caractérisé en ce qu'il contient à titre de conservateur de 0,005 à 0,5% du composé selon l'une quelconque des revendications 1 à 4.

11. Produit alimentaire, caractérisé en ce qu'il contient à titre de conservateur de 0,005 à 0,5% du composé selon l'une quelconque des revendications 1 à 4.

**Revendication pour l'Etat Contractant suivant : GR**

1. Composé de formule :

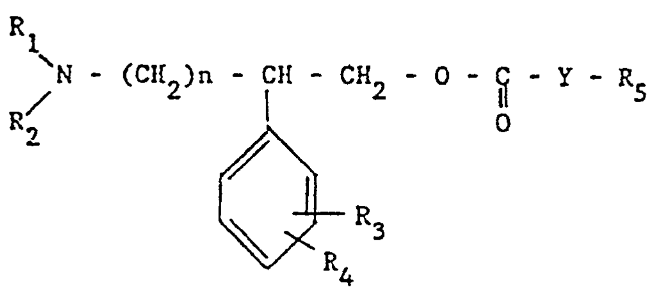

(I)

dans laquelle :

— n est un nombre entier compris entre 2 et 10 ;

— $R_1$ et $R_2$ sont identiques ou différents et représentent un cycloalkyle de 3 à 6 atomes de carbone,un alkyle comportant de 1 à 6 atomes de carbone non substitué ou substitué par un groupement phényle ou benzyle ;

ou $R_1$ et $R_2$ forment ensemble avec l'atome d'azote auquel ils sont liés un hétérocycle choisi parmi les groupes pyrrolidinyl-1, pipéridino, azépinyl-1, hexaméthylèneimino, méthyl-4 pipéridino, benzyl-4 pipéridino, phényl-4 pipéridino, tétrahydro-1,2,3,4 isoquinolyl-2, morpholino, imidazolyl-1 ;

— $R_3$ représente un hydrogène, un halogène, un méthyle ou un phényle ;

— $R_4$ représente un hydrogène, un halogène ou un méthyle ;

ou $R_3$ et $R_4$ pris ensemble avec le noyau benzénique auquel ils sont liés constituent un groupement naphtyl-1 ou naphtyl-2 ;

— Y représente une liaison directe, un groupement méthylèneoxy, un groupement méthylènethio ou un groupement vinylène ;

— $R_5$ représente un alkyle comportant de 5 à 18 atomes de carbone, un cycloalkyle comportant de 3 à 8 atomes de carbone, un groupe adamantyle, naphtyl-1, naphtyl-2 ou phényle non substitué ou substitué par un ou 2 substituants choisis parmi les atomes d'halogènes, le groupe trifluorométhyle, le groupe nitro ou le groupe phényle ;

ainsi que leurs sels avec les acides minéraux ou organiques.

2. Composé selon la revendication 1, caractérisé en ce que $R_3$ représente un hydrogène, un halogène, un méthyle ou un phényle et $R_4$ représente un hydrogène, un halogène ou un méthyle.

3. Composé selon la revendication 1, caractérisé en ce que $R_3$ et $R_4$ pris ensemble avec le noyau benzénique auquel ils sont liés constituent un groupement naphtyl-1 ou naphtyl-2.

4. Le dichloro-2,4 benzoate de diméthylamino-5 (naphtyl-1)-2 pentyle et ses sels.

5. Procédé pour la préparation d'un composé selon l'une quelconque des revendications 1 à 4, caractérisé en ce que l'on estérifie l'aminoalcool de formule :

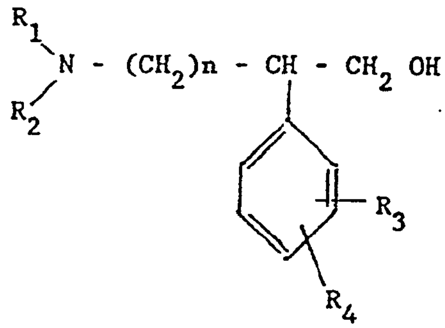

(II)

dans laquelle $R_1$, $R_2$, $R_3$, $R_4$ et n sont tels que définis dans la revendication 1, ou un de ses sels avec un acide minéral ou organique, par un acide ou un halogénure d'acide de formule :

$$Z - \underset{\underset{O}{\|}}{C} - Y - R_5 \qquad \text{(III)}$$

dans laquelle Y et $R_5$ sont tels que définis dans la revendication 1 et Z représente le groupement OH ou un halogénure.

6. Utilisation d'un composé selon l'une quelconque des revendications 1 à 4, comme antiseptique, désinfectant, conservateur dans des préparations désinfectante, cosmétique ou alimentaire.

7. Composition présentant une activité antimicrobienne et désinfectante, caractérisée en ce qu'elle contient de 0,01 à 5% du composé selon l'une quelconque des revendications 1 à 4.

8. Composition désinfectante pour surfaces inertes, caractérisée en ce qu'elle contient de 0,1 à 4% du composé selon l'une quelconque des revendications 1 à 4.

9. Composition caractérisée en ce qu'elle contient à titre de conservateur de 0,005 à 0,5% du composé selon l'une quelconque des revendications 1 à 4.

10. Produit cosmétique, caractérisé en ce qu'il contient à titre de conservateur de 0,005 à 0,5% du composé selon l'une quelconque des revendications 1 à 4.

11. Produit alimentaire, caractérisé en ce qu'il contient à titre de conservateur de 0,005 à 0,5% du composé selon l'une quelconque des revendications 1 à 4.

## Claims

### Claims for the following Contracting States : AT, BE, CH, DE, FR, GB, IT, LI, LU, NL, SE

1. A compound of the formula

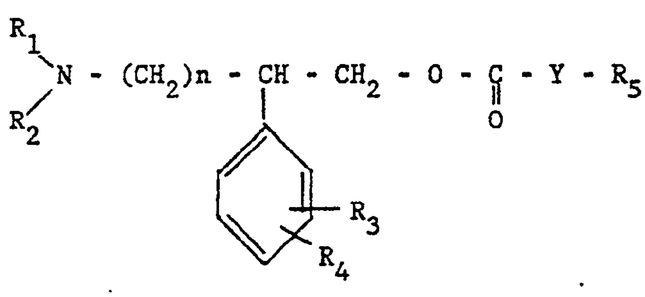

(I)

in which :

— n is an integer between 2 and 10 ;

— $R_1$ and $R_2$ are identical or different and represent a cycloalkyl having 3 to 6 carbon atoms or an alkyl containing from 1 to 6 carbon atoms which is unsubstituted or substituted by a phenyl or benzyl group ; or $R_1$ and $R_2$, together with the nitrogen atom to which they are bonded, form a heterocycle selected from pyrrolidin-1-yl, piperidino, azepin-1-yl, hexamethyleneimino, 4-methylpiperidino, 4-benzylpipelidino, 4-phenylpiperidino, 1,2,3,4-tetrahydroisoquinol-2-yl, morpholino and imidazol-1-yl groups ;

— $R_3$ represents a hydrogen, a halogen, a methyl or a phenyl ;

— $R_4$ represents a hydrogen, a halogen or a methyl ; or $R_3$ and $R_4$, taken together with the benzene ring to which they are bonded, form a naphth-1-yl or naphth-2-yl group ;

— Y represents a direct bond, a methyleneoxy group, a methylenethio group or a vinylene group ;

— $R_5$ represents an alkyl containing from 5 to 18 carbon atoms, a cycloalkyl containing from 3 to 8 carbon atoms, an adamantyl, naphth-1-yl or naphth-2-yl group, an unsubstituted phenyl group or a phenyl group substituted by one or 2 substituents selected from halogen atoms, the trifluoromethyl group, the nitro group and the phenyl group ;

and its salts with mineral or organic acids.

2. A compound according to claim 1, characterized in that $R_3$ represents a hydrogen, a halogen, a methyl or a phenyl and $R_4$ represents a hydrogen, a halogen or a methyl.

3. A compound according to claim 1, characterized in that $R_3$ and $R_4$, taken together with the benzene ring to which they are bonded, form a naphth-1-yl or naphth-2-yl group.

4. 5-Dimethylamino-2-(naphth-1-yl)pentyl 2,4-dichlorobenzoate and its salts.

5. A process for the preparation of a compound according to any one of claims 1 to 4, characterized in that it comprises esterifying the amino alcohol of the formula :

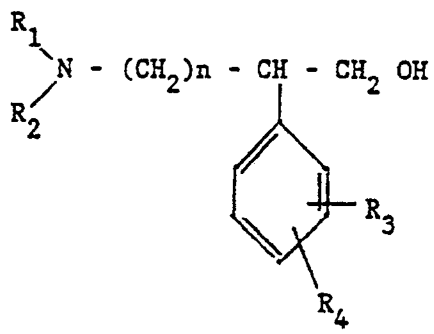

(II)

in which $R_1$, $R_2$, $R_3$, $R_4$ and n are as defined in claim 1, or one of its salts with a mineral or organic acid, with an acid or an acid halide of the formula :

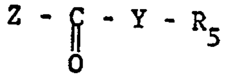

(III)

in which Y and $R_5$ are as defined in claim 1 and Z represents the OH group or a halide.

6. Use of a compound according to any one of claims 1 to 4, as antiseptic, disinfectant or preservative in pharmaceutical, disinfectant, cosmetic or food preparations.

7. A pharmaceutical composition having an antimicrobial and disinfectant activity, characterized in that it contains from 0.01 to 5% of the compound according to any one of claims 1 to 4.

8. A disinfectant composition for inert surfaces, characterized in that it contains from 0.1 to 4% of the compound according to any one of claims 1 to 4.

9. A pharmaceutical composition, characterized in that it contains from 0.005 to 0.5% of the compound according to any one of claims 1 to 4 as a preservative.

10. A cosmetic product, characterized in that it contains from 0.005 to 0.5% of the compound according to any one of claims 1 to 4 as a preservative.

11. A foodstuff, characterized in that it contains from 0.005 to 0.5% of the compound according to any one of claims 1 to 4 as a preservative.

**Claims for the following Contracting State : ES**

1. A process for the preparation of aromatic derivatives corresponding to the formula :

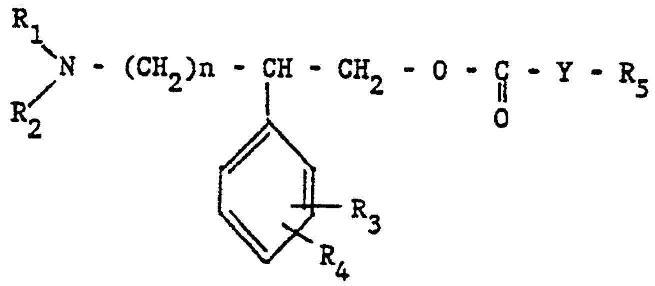

(I)

in which :

— n is an integer between 2 and 10 ;

— $R_1$ and $R_2$ are identical or different and represent a cycloalkyl having 3 to 6 carbon atoms or an alkyl containing from 1 to 6 carbon atoms which is unsubstituted or substituted by a phenyl or benzyl group ; or

$R_1$ and $R_2$, together with the nitrogen atom to which they are bonded, form a heterocycle selected from pyrrolidin-1-yl, piperidino, azepin-1-yl, hexamethyleneimino, 4-methylpiperidino, 4-benzylpipe-ridino, 4-phenylpiperidino, 1,2,3,4-tetrahydroisoquinol-2-yl, morpholino and imidazol-1-yl groups ;

— $R_3$ represents a hydrogen, a halogen, a methyl or a phenyl ;

— $R_4$ represents a hydrogen, a halogen or a methyl ; or $R_3$ and $R_4$, taken together with the benzene ring to which they are bonded, form a naphth-1-yl or naphth-2-yl group ;

— Y represents a direct bond, a methyleneoxy group, a methylenethio group or a vinylene group ;

— $R_5$ represents an alkyl containing from 5 to 18 carbon atoms, a cycloalkyl containing from 3 to 8 carbon atoms, an adamantyl, naphth-1-yl or naphth-2-yl group, an unsubstituted phenyl group or a phenyl group substituted by one or 2 substituents selected from halogen atoms, the trifluoromethyl group, the nitro group and the phenyl group ; characterized in that it comprises esterifying the amino alcohol of the formula :

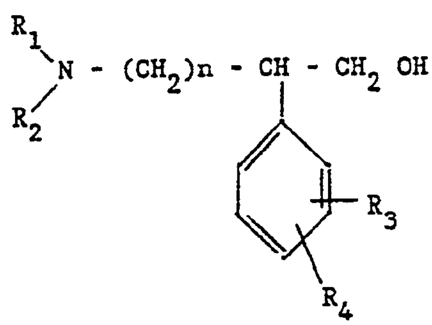

(II)

in which $R_1$, $R_2$, $R_3$, $R_4$ and n are as above defined, or one of its salts with a mineral or organic acid, with an acid or an acid halide of the formula :

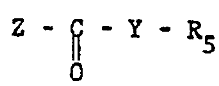

(III)

in which Y and $R_5$ are as above defined and Z represents the OH group or a halide.

2. A process according to claim 1, characterized in that an aminoalcohol of formula (II) is used in which $R_3$ represents a hydrogen, a halogen, a methyl or a phenyl and $R_4$ represents a hydrogen, a halogen or a methyl.

3. A process according to claim 1, characterized in that an aminoalcohol of formula II is used in which $R_3$ and $R_4$, taken together with the benzene ring to which they are bonded, form a naphth-1-yl or naphth-2-yl group.

4. A process according to claim 1, characterized in that a compound of formula II is used as amino alcohol, in which $R_1$ and $R_2$ are the methyl group, n is 3 and $R_3$ and $R_4$ form with the benzene group, to which they are

bonded, the naphth-1-yl group and in that the said amino alcohol is reacted with a compound of formula III in which Z is as defined in claim 1, Y is a direct bond and $R_5$ is the 2,4 dichlorophenyl group.

5. Use of a compound of formula (I) obtained by the process according to any one of claims 1 to 4, as antiseptic, disinfectant, preservative in disinfectant, cosmetic or food preparations.

6. Use of a compound of formula (I) obtained by the process according to any one of claims 1 to 4, in association with a pharmaceutically acceptable carrier for the preparation of pharmaceutical compositions.

7. Use according to claim 6, for the preparation of pharmaceutical compositions having an antimicrobial and disinfectant activity, which contain from 0.01 to 5% of the compound of formula (I).

8. Disinfectant composition for inert surfaces, characterized in that it contains from 0.1 to 4% of the compound according to any one of claims 1 to 4.

9. Composition, characterized in that it contains from 0.005 to 0.5% of the compound according to any one of claims 1 to 4 as a preservative.

10. A cosmetic product, characterized in that it contains from 0.005 to 0.5% of the compound according to any one of claims 1 to 4 as a preservative.

11. A foodstuff, characterized in that it contains from 0.005 to 0.5% of the compound according to any one of claims 1 to 4 as a preservative.

**Claims for the following Contracting State : GR**

1. A compound of the formula :

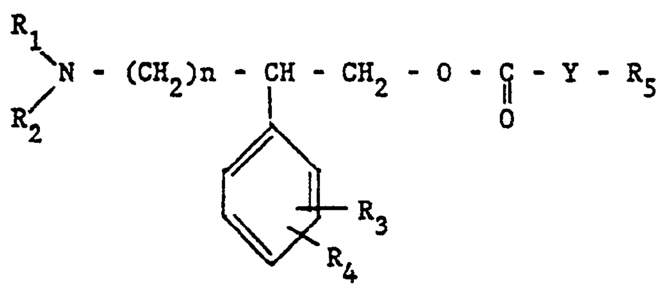

(I)

in which :

— n is an integer between 2 and 10 ;

— $R_1$ and $R_2$ are identical or different and represent a cycloalkyl having 3 to 6 carbon atoms or an alkyl containing from 1 to 6 carbon atoms which is unsubstituted or substituted by a phenyl or benzyl group ; or $R_1$ and $R_2$, together with the nitrogen atom to which they are bonded, form a heterocycle selected from pyrrolidin-1-yl, piperidino, azepin-1-yl, hexamethyleneimino, 4-methylpiperidino, 4-benzylpiperidino, 4-phenylpiperidino, 1,2,3,4-tetrahydroisoquinol-2-yl, morpholino and imidazol-1-yl groups ;

— $R_3$ represents a hydrogen, a halogen, a methyl or a phenyl ;

— $R_4$ represents a hydrogen, a halogen or a methyl ; or $R_3$ and $R_4$, taken together with the benzene ring to which they are bonded, form a naphth-1-yl or naphth-2-yl group ;

— Y represents a direct bond, a methyleneoxy group, a methylenethio group or a vinylene group ;

— $R_5$ represents an alkyl containing from 5 to 18 carbon atoms, a cycloalkyl containing from 3 to 8 carbon atoms, an adamantyl, naphth-1-yl or naphth-2-yl group, an unsubstituted phenyl group or a phenyl group substituted by one or 2 substituents selected from halogen atoms, the trifluoromethyl group, the nitro group and the phenyl group ;

and its salts with mineral or organic acids.

2. A compound according to claim 1, characterized in that $R_3$ represents a hydrogen, a halogen, a methyl or a phenyl and $R_4$ represents a hydrogen, a halogen or a methyl.

3. A compound according to claim 1, characterized in that $R_3$ and $R_4$, taken together with the benzene ring to which they are bonded, form a naphth-1-yl or naphth-2-yl group.

4. 5-Dimethylamino-2-(naphth-1-yl)pentyl 2,4-dichlorobenzoate and its salts.

5. A process for the preparation of a compound according to any one of claims 1 to 4, characterized in that it comprises esterifying the amino alcohol of the formula :

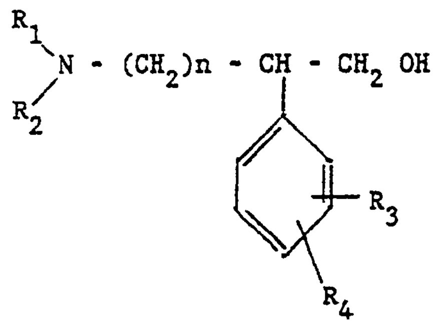

(II)

in which $R_1$, $R_2$, $R_3$, $R_4$ and n are as defined in claim 1, or one of its salts with a mineral or organic acid, with an acid or an acid halide of the formula :

$$Z - \underset{\overset{\|}{O}}{C} - Y - R_5 \qquad (III)$$

in which Y and $R_5$ are as defined in claim 1 and Z represents the OH group or a halide.

6. Use of a compound according to any one of claims 1 to 4, as antiseptic, disinfectant or preservative in disinfectant, cosmetic or food preparations.

7. A composition having an antimicrobial and disinfectant activity, characterized in that it contains from 0.01 to 5% of the compound according to any one of claims 1 to 4.

8. A disinfectant composition for inert surfaces, characterized in that it contains from 0.1 to 4% of the compound according to any one of claims 1 to 4.

9. A composition, characterized in that it contains from 0.005 to 0.5% of the compound according to any one of claims 1 to 4 as a preservative.

10. A cosmetic product, characterized in that it contains from 0.005 to 0.5% of the compound according to any one of claims 1 to 4 as a preservative.

11. A foodstuff, characterized in that it contains from 0.005 to 0.5% of the compound according to any one of claims 1 to 4 as a preservative.

## Patentansprüche

### Patentansprüche für folgenden Vertragsstaaten : AT, BE, CH, DE, FR, GB, IT, LI, LU, NL, SE

1. Verbindung der Formel :

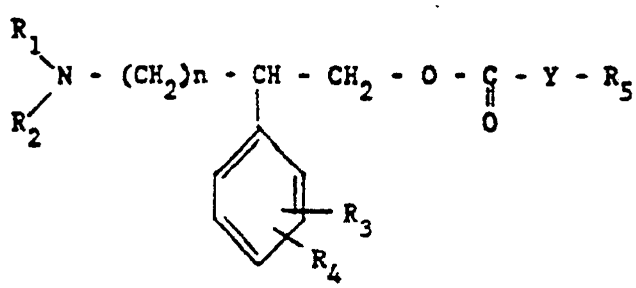

(I)

in welcher :

— n eine ganze Zahl zwischen 2 und 10 ist ;

— $R_1$ und $R_2$ gleich oder verschieden sind und Cycloalkyl mit 3 bis 6 Kohlenstoffatomen, Alkyl mit 1 bis 6 Kohlenstoffatomen, nicht substituiert oder durch eine Phenyl- oder Benzylgruppe substituiert, darstellen ; oder $R_1$ und $R_2$, zusammen mit dem Stickstoffatom, an das sie gebunden sind, einen Heterocyclus bilden, ausgewählt aus den Gruppen Pyrrolidin-1-yl, Piperidino, Azepin-1-yl, Hexamethylenimino, 4-Methyl-piperidino, 4-Benzyl-piperidino, 4-Phenyl-piperidino, 1,2,3,4-Tetrahydro-isochinol-2-yl, Morpholino, Imidazol-

1-yl ;
— $R_3$ Wasserstoff, Halogen, Methyl oder Phenyl darstellt ;
— $R_4$ Wasserstoff, Halogen oder Methyl darstellt ;
oder $R_3$ und $R_4$, zusammen mit dem Benzolkern, an den sie gebunden sind, eine 1-Naphthyl- oder 2-Naphthylgruppe bilden ;
— Y eine direkte Bindung, eine Methylenoxygruppe, eine Methylenthiogruppe oder eine Vinylengruppe bedeutet ;
— $R_5$ ein Alkyl mit 5 bis 18 Kohlenstoffatomen, ein Cycloalkyl mit 3 bis 8 Kohlenstoffatomen, eine Adamantylgruppe, 1-Naphthyl, 2-Naphthyl oder Phenyl, nicht substituiert oder durch einen oder zwei Substituenten, ausgewählt aus Halogenatomen, Trifluormethyl, einer Nitrogruppe oder Phenyl, substituiert, darstellt ;
sowie ihre Salze mit Mineral- oder organischen Säuren.

2. Verbindung nach Anspruch 1, dadurch gekennzeichnet, daß $R_3$ Wasserstoff, Halogen, Methyl oder Phenyl darstellt und $R_4$ Wasserstoff, Halogen oder Methyl bedeutet.

3. Verbindung nach Anspruch 1, dadurch gekennzeichnet, daß $R_3$ und $R_4$, zusammen mit dem Benzolkern, an den sie gebunden sind, eine 1-Naphthyl- oder 2-Naphthylgruppe bilden.

4. 5-Dimethylamino-2-(naphth-1-yl)-pentyl-2,4-Dichlorbenzoat und seine Salze.

5. Verfahren zur Herstellung einer Verbindung nach einem der Ansprüche 1 bis 4, dadurch gekennzeichnet, daß man einen Aminoalkohol der Formel :

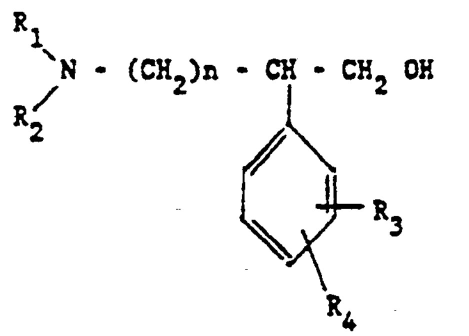

(II)

in welcher $R_1$, $R_2$, $R_3$, $R_4$ und n wie in Anspruch 1 definiert sind, oder eines seiner Salze mit einer Mineral- oder organischen Säure, mit einer Säure oder einem Säurehalogenid der Formel :

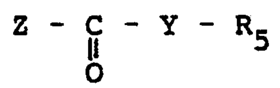

(III),

worin Y und $R_5$ wie in Anspruch 1 definiert sind, und Z für die Gruppe OH oder ein Halogenid steht, verestert.

6. Verwendung einer Verbindung nach einem der Ansprüche 1 bis 4 als Antiseptikum, Desinfektionsmittel oder Konservierungsmittel in pharmazeutischen, desinfizierenden, kosmetischen oder alimentären Präparationen.

7. Pharmazeutische Zusammensetzung mit antimikrobieller und desinfizierender Wirkung, dadurch gekennzeichnet, daß sie 0,01 bis 5% der Verbindung nach einem der Ansprüche 1 bis 4 enthält.

8. Desinfizierende Zusammensetzung für inerte Oberflächen, dadurch gekennzeichnet, daß sie 0,1 bis 4% der Verbindung nach einem der Ansprüche 1 bis 4 enthält.

9. Pharmazeutische Zusammensetzung, dadurch gekennzeichnet, daß sie als Konservierungsmittel 0,005 bis 0,5% der Verbindung nach einem der Ansprüche 1 bis 4 enthält.

10. Kosmetisches Produkt, dadurch gekennzeichnet, daß es als Konservierungsmittel 0,005 bis 0,5% der Verbindung nach einem der Ansprüche 1 bis 4 enthält.

11. Nahrungsmittel, dadurch gekennzeichnet, daß es als Konservierungsmittel 0,005 bis 0,5% der Verbindung nach einem der Ansprüche 1 bis 4 enthält.

**Patentansprüche für folgenden Vertragsstaat : ES**

1. Verfahren zur Herstellung von aromatischen Derivaten der Formel :

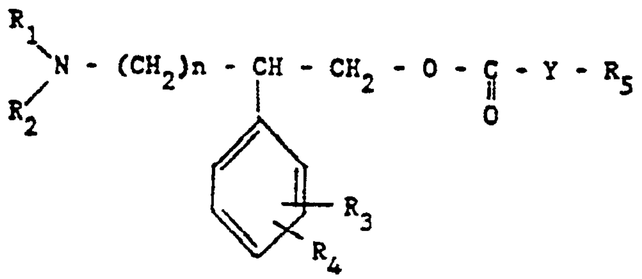

(I)

in welcher :

— n eine ganze Zahl zwischen 2 und 10 ist ;

— $R_1$ und $R_2$ gleich oder verschieden sind und Cycloalkyl mit 3 bis 6 Kohlenstoffatomen, Alkyl mit 1 bis 6 Kohlenstoffatomen, nicht substituiert oder durch eine Phenyl- oder Benzylgruppe substituiert, darstellen ; oder $R_1$ und $R_2$, zusammen mit dem Stickstoffatom, an das sie gebunden sind, einen Heterocyclus bilden, ausgewählt aus den Gruppen Pyrrolidin-1-yl, Piperidino, Azepin-1-yl, Hexamethylenimino, 4-Methyl-piperidino, 4-Benzyl-piperidino, 4-Phenyl-piperidino, 1,2,3,4-Tetrahydro-isochinol-2-yl, Morpholino, Imidazol-1-yl ;

— $R_3$ Wasserstoff, Halogen, Methyl oder Phenyl darstellt ;

— $R_4$ Wasserstoff, Halogen oder Methyl darstellt ;

oder $R_3$ und $R_4$, zusammen mit dem Benzolkern, an den sie gebunden sind, eine 1-Naphthyl- oder 2-Naphthylgruppe bilden ;

— Y eine direkte Bindung, eine Methylenoxygruppe, eine Methylenthiogruppe oder eine Vinylengruppe bedeutet ;

— $R_5$ ein Alkyl mit 5 bis 18 Kohlenstoffatomen, ein Cycloalkyl mit 3 bis 8 Kohlenstoffatomen, eine Adamantylgruppe, 1-Naphthyl, 2-Naphthyl oder Phenyl, nicht substituiert oder substituiert durch einen oder zwei Substituenten, ausgewählt aus Halogenatomen, Trifluormethyl, einer Nitrogruppe oder Phenyl, darstellt ; dadurch gekennzeichnet, daß man einen Aminoalkohol der Formel :

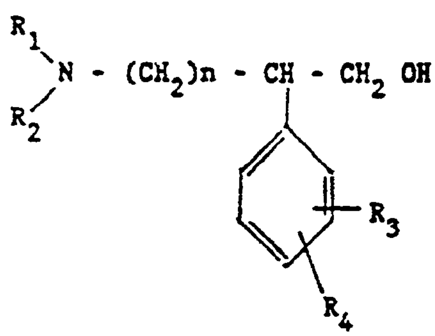

(II)

in welcher $R_1$, $R_2$, $R_3$, $R_4$ und n wie oben definiert sind, oder eines seiner Salze mit einer Mineral- oder organischen Säure, mit einer Säure oder einem Säurehalogenid der Formel :

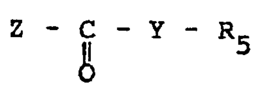

(III),

worin Y und $R_5$ wie oben definiert sind, und Z für die Gruppe OH oder ein Halogenid steht, verestert.

2. Verfahren nach Anspruch 1, dadurch gekennzeichnet, daß man einen Aminoalkohol der Formel (II), in welcher $R_3$ Wasserstoff, Halogen, Methyl oder Phenyl darstellt und $R_4$ Wasserstoff, Halogen oder Methyl bedeutet, verwendet.

3. Verfahren nach Anspruch 1, dadurch gekennzeichnet, daß man einen Aminoalkohol der Formel (II), in welcher $R_3$ und $R_4$, zusammen mit dem Benzolkern, an den sie gebunden sind, eine 1-Naphthyl- oder 2-Naphthylgruppe bilden, verwendet.

4. Verfahren nach Anspruch 1, dadurch gekennzeichnet, daß man als Aminoalkohol die Verbindung der Formel II verwendet, in welcher $R_1$ und $R_2$ Methyl sind, n für 3 steht und $R_3$ und $R_4$ mit der Benzolgruppe, an die sie gebunden sind, 1-Naphthyl bilden, und daß man den Aminoalkohol mit einer Verbindung der Formel III, in welcher Z wie in Anspruch 1 definiert ist, Y eine direkte Bindung darstellt und $R_5$ für die Gruppe 2,4-Dichlorphenyl steht, reagieren läßt.

5. Verwendung einer Verbindung der Formel (I), erhalten durch das Verfahren nach einem der Ansprüche 1 bis 4, als Antiseptikum, Desinfektionsmittel oder Konservierungsmittel in desinfizierenden, kosmetischen oder alimentären Präparationen.

6. Verwendung einer Verbindung der Formel (I), erhalten durch das Verfahren nach einem der Ansprüche 1 bis 4, in Kombination mit einem pharmazeutisch akzeptablen Vehikel zur Herstellung von pharmazeutischen Zusammensetzungen.

7. Verwendung nach Anspruch 6 zur Herstellung von pharmazeutischen Zusammensetzungen mit antimikrobieller und desinfizierender Aktivität, welche 0,01 bis 5% der Verbindung der Formel I enthalten.

8. Desinfizierende Zusammensetzung für inerte Oberflächen, dadurch gekennzeichnet, daß sie 0,1 bis 4% der Verbindung nach einem der Ansprüche 1 bis 4 enthält.

9. Zusammensetzung, dadurch gekennzeichnet, daß sie als Konservierungsmittel 0,005 bis 0,5% der Verbindung nach einem der Ansprüche 1 bis 4 enthält.

10. Kosmetisches Produkt, dadurch gekennzeichnet, daß es als Konservierungsmittel 0,005 bis 0,5% der Verbindung nach einem der Ansprüche 1 bis 4 enthält.

11. Nahrungsmittel, dadurch gekennzeichnet, daß es als Konservierungsmittel 0,005 bis 0,5% der Verbindung nach einem der Ansprüche 1 bis 4 enthält.

**Patentansprüche für folgenden Vertragsstaat : GR**

1. Verbindung der Formel :

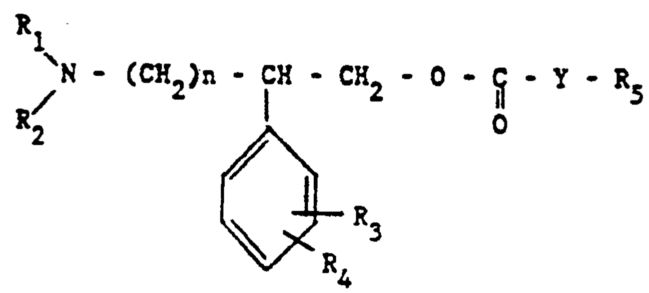

(I)

in welcher :

— n eine ganze Zahl zwischen 2 und 10 ist ;

— $R_1$ und $R_2$ gleich oder verschieden sind und Cycloalkyl mit 3 bis 6 Kohlenstoffatomen, Alkyl mit 1 bis 6 Kohlenstoffatomen, nicht substituiert oder durch eine Phenyl- oder Benzylgruppe substituiert, darstellen ; oder $R_1$ und $R_2$, zusammen mit dem Stickstoffatom, an das sie gebunden sind, einen Heterocyclus bilden, ausgewählt aus den Gruppen Pyrrolidin-1-yl, Piperidino, Azepin-1-yl, Hexamethylenimino, 4-Methyl-piperidino, 4-Benzyl-piperidino, 4-Phenyl-piperidino, 1,2,3,4-Tetrahydroisochinol-2-yl, Morpholino, Imidazol-1-yl ;

— $R_3$ Wasserstoff, Halogen, Methyl oder Phenyl darstellt ;

— $R_4$ Wasserstoff, Halogen oder Methyl darstellt ;

oder $R_3$ und $R_4$, zusammen mit dem Benzolkern, an den sie gebunden sind, eine 1-Naphthyl- oder 2-Naphthylgruppe bilden ;

— Y eine direkte Bindung, eine Methylenoxygruppe, eine Methylenthiogruppe oder eine Vinylengruppe bedeutet ;

— $R_5$ ein Alkyl mit 5 bis 18 Kohlenstoffatomen, ein Cyclo-alkyl mit 3 bis 8 Kohlenstoffatomen, eine Adamantylgruppe, 1-Naphthyl, 2-Naphthyl oder Phenyl, nicht substituiert oder substituiert durch einen oder zwei Substituenten, ausgewählt aus Halogenatomen, Trifluormethyl, einer Nitrogruppe oder Phenyl, darstellt ;

sowie ihre Salze mit Mineral- oder organischen Säuren.

2. Verbindung nach Anspruch 1, dadurch gekennzeichnet, daß $R_3$ Wasserstoff, Halogen, Methyl oder Phe-

nyl darstellt und $R_4$ Wasserstoff, Halogen oder Methyl bedeutet.

3. Verbindung nach Anspruch 1, dadurch gekennzeichnet, daß $R_3$ und $R_4$, zusammen mit dem Benzolkern, an den sie gebunden sind, eine 1-Naphthyl- oder 2-Naphthylgruppe bilden.

4. 5-Dimethylamino-2-(naphth-1-yl)-pentyl-2,4-Dichlorbenzoat und seine Salze.

5. Verfahren zur Herstellung einer Verbindung nach einem der Ansprüche 1 bis 4, dadurch gekennzeichnet, daß man einen Aminoalkohol der Formel :

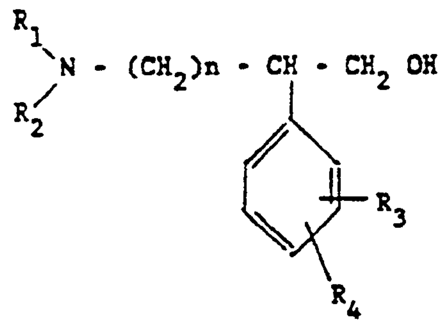

(II)

in welcher $R_1$, $R_2$, $R_3$, $R_4$ und n wie in Anspruch 1 definiert sind, oder eines seiner Salze mit einer Mineral- oder organischen Säure, mit einer Säure oder einem Säurehalogenid der Formel :

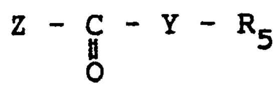

(III),

worin Y und $R_5$ wie in Anspruch 1 definiert sind, und Z für die Gruppe OH oder ein Halogenid steht, verestert.

6. Verwendung einer Verbindung nach einem der Ansprüche 1 bis 4 als Antiseptikum, Desinfektionsmittel oder Konservierungsmittel in pharmazeutischen, desinfizierenden, kosmetischen oder alimentären Präparationen.

7. Zusammensetzung mit antimikrobieller und desinfizierender Aktivität, dadurch gekennzeichnet, daß sie 0,01 bis 5% der Verbindung nach einem der Ansprüche 1 bis 4 enthält.

8. Desinfizierende Zusammensetzung für inerte Oberflächen, dadurch gekennzeichnet, daß sie 0,1 bis 4% der Verbindung nach einem der Ansprüche 1 bis 4 enthält.

9. Zusammensetzung, dadurch gekennzeichnet, daß sie als Konservierungsmittel 0,005 bis 0,5% der Verbindung nach einem der Ansprüche 1 bis 4 enthält.

10. Kosmetisches Produkt, dadurch gekennzeichnet, daß es als Konservierungsmittel 0,005 bis 0,5% der Verbindung nach einem der Ansprüche 1 bis 4 enthält.

11. Nahrungsmittel, dadurch gekennzeichnet, daß es als Konservierungsmittel 0,005 bis 0,5% der Verbindung nach einem der Ansprüche 1 bis 4 enthält.